(19) Europäisches Patentamt
European Patent Office
Office européen des brevets

(11) EP 1 226 113 B1

(12) **FASCICULE DE BREVET EUROPEEN**

(45) Date de publication et mention de la délivrance du brevet:
**09.11.2005 Bulletin 2005/45**

(21) Numéro de dépôt: **00964569.8**

(22) Date de dépôt: **09.10.2000**

(51) Int Cl.[7]: **C07C 233/05**, A61K 7/46, C07C 233/09, C07C 233/65

(86) Numéro de dépôt international:
**PCT/IB2000/001454**

(87) Numéro de publication internationale:
**WO 2001/028980 (26.04.2001 Gazette 2001/17)**

(54) **ESTERS COMPORTANT UNE FONCTION CARBAMOYLE SECONDAIRE ET LEUR UTILISATION EN TANT QUE PRECURSEURS D'ALCOOLS ODORANTS**

ESTER MIT EINER SEKUNDÄREN CARBAMOYLGRUPPE UND DEREN VERWENDUNG ALS AUSGANGSSTOFFE VON DUFTENDEN ALKOHOLEN

ESTERS COMPRISING A SECONDARY CARBAMOYL FUNCTION AND THEIR USE AS ODORANT ALCOHOL PRECURSORS

(84) Etats contractants désignés:
**AT BE CH CY DE DK ES FI FR GB GR IE IT LI LU MC NL PT SE**

(30) Priorité: **18.10.1999 CH 189499**

(43) Date de publication de la demande:
**31.07.2002 Bulletin 2002/31**

(73) Titulaire: **Firmenich S.A.**
**1211 Geneva 8 (CH)**

(72) Inventeur: **FREROT, Eric**
**F-74100 Ville la Grand (FR)**

(74) Mandataire:
**Salvaterra-Garcia, Maria de Lurdes et al**
**Firmenich SA,**
**1, route des Jeunes**
**1211 Genève 8 (CH)**

(56) Documents cités:
**EP-A- 0 085 880**
**WO-A-98/47478**
**JP-A- 3 213 850**
**JP-A- 7 261 345**
**US-A- 2 783 206**
**US-A- 2 885 319**
**US-A- 3 170 889**
**US-A- 4 362 870**
**US-A- 4 528 133**

- **WANG, SHAOMENG ET AL: "The Discovery of Novel, Structurally Diverse Protein Kinase C Agonists through Computer 3D-Database Pharmacophore Search. Molecular Modeling Studies" J. MED. CHEM. (1994), 37(26), 4479-89 , XP002159623**
- **TANCHUK, YU. V. ET AL: "Reaction of maleic acid diesters with ethylene- and hexamethylenediamines, and monoethanolamine" ZH. ORG. KHIM. (1978), 14(11), 2252-8 , XP000981636**
- **TANCHUK, YU. V. ET AL: "Reaction of maleic acid esters with ethylenediamine" UKR. KHIM. ZH. (RUSS. ED.) (1976), 42(4), 390-4 , XP000981724**
- **VASILEVSKAYA, T. N. ET AL: "Alkylation of phthalimide by alkyl bromides under phase-transfer catalysis conditions" ZH. PRIKL. KHIM. (LENINGRAD) (1987), 60(1), 233-6 , XP002159624**
- **CHEMICAL ABSTRACTS, vol. 84, no. 7, 16 février 1976 (1976-02-16) Columbus, Ohio, US; abstract no. 43697, TAKEMATSU, TETSUO ET AL: "N-(.alpha.-naphthyl) phthalamates" XP002159625 & Chem. Abs. 9th Collective Index & JP 49 034977 B (MITSUBISHI CHEMICAL INDUSTRIES CO., LTD., JAPAN) 19 septembre 1974 (1974-09-19)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

### Domaine technique

La présente invention a trait au domaine de la parfumerie. Elle concerne plus particulièrement des esters susceptibles de libérer un alcool odorant et comportant, en proximité, une fonction carbamoyle nouveaux qui facilite la libération dudit l'alcool odorat.

### Technique antérieure

Le domaine de la parfumerie témoigne d'un intérêt particulier pour les composés capables de prolonger un effet odorant sur une certaine période de temps, en particulier pour pallier les problèmes rencontrés lors de l'utilisation d'ingrédients parfumants trop volatiles. On connaît notamment du brevet US 5,649,979 des composés qui, dans certaines conditions d'activation telles que la lumière, la chaleur, la présence d'enzymes, notamment de lipases, sont susceptibles de libérer une molécule odorante et ce, sur une période de temps étalée. De tels composés peuvent être utiles dans diverses applications. Le lavage de textiles est en particulier un domaine dans lequel on recherche toujours de nouveaux moyens permettant de percevoir l'effet de substances parfumantes pendant une certaine période après les opérations de lavage et de séchage. En effet beaucoup de substances ayant des odeurs particulièrement adaptées pour ce type d'application sont connues pour manquer de ténacité sur le linge, ou ne pas rester sur le linge au moment des rinçages, de sorte que leur effet parfumant n'est perçu que brièvement et peu intensément. Etant donnée l'importance de ce type d'application dans l'industrie de la parfumerie, l'activité de recherche dans ce domaine reste soutenue, notamment dans le but de trouver de nouvelles solutions toujours plus performantes pour remédier aux problèmes susmentionnés.

### Exposé de l'invention

Or, nous avons découvert de façon surprenante l'existence de nouveaux esters comportant une fonction carbamoyle non substituée ou mono-substituée, susceptibles de libérer de façon contrôlée et efficace une molécule, en particulier un alcool odorant, sans l'aide d'un activateur tel que décrit dans l'art antérieur. Ces composés présentent en outre, de façon tout à fait imprévue, une excellente ténacité, notamment sur le linge, ce qui en fait des précurseurs très utiles en particulier pour des applications liées au domaine de la parfumerie fonctionnelle. En effet, des molécules odorantes présentes en tant que telles dans des produits tels que les lessives ou détergents sont en général peu tenaces et sont par conséquent souvent éliminés dans les eaux de rinçage lors d'un lavage en machine par exemple. A l'inverse, les composés de l'invention, grâce à leur ténacité et à la libération contrôlée de l'alcool odorant, permettent d'impartir au linge une odeur et une fraîcheur qui perdurera après les opérations de rinçage et de séchage.

La présente invention concerne une composition parfumant ou article parfumé contenant des ingrédiants parfumants, solvants et, ou, adjuvants couramment utilisés pour la préparation de parfums et, en tant qu'ingrédient actif, un composé de formule

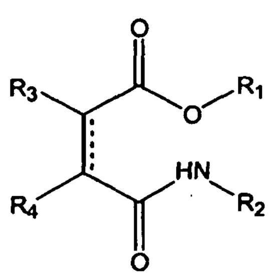

ou

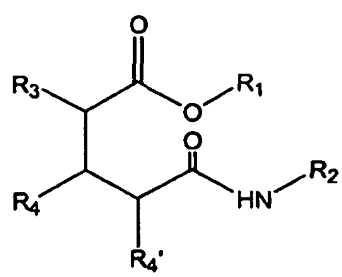

(I)

dans laquelle la ligne pointillée indique l'emplacement d'une liaison simple ou double ; $R_1$ représente un radical dérivé d'un alcool odorant de formule $R_1OH$ ; $R_2$ représente un atome d'hydrogène, un radical d'hydrocarbure linéaire ou ramifié, saturé ou insaturé, de $C_1$ à $C_{30}$, ou un cycle aliphatique ou aromatique ayant 5 ou 6 atomes de carbone, ce radical $R_2$ pouvant comporter des hétéroatomes d'oxygène, de soufre ou d'azote, en particulier des fonctions ammonium quaternaires ; les symboles $R_3$, $R_4$ et $R_4'$ pris indépendamment, représentent un atome d'hydrogène, un radical d'hydrocarbure linéaire ou ramifié, saturé ou insaturé, le cas échéant substitué de $C_1$ à $C_{20}$ pouvant comporter un ou

plusieurs hétéroatomes ou, lorsque pris ensemble avec les atomes de carbone auxquels ils sont liés, peuvent former des monocycles, bicycles ou tricycles aromatiques ou aliphatiques, les radicaux $R_3$, $R_4$ et $R_4$' pouvant comprendre des groupes fonctionnels de type ester et carbamoyle de façon à libérer plusieurs molécules d'alcool odorant $R_1$-OH pour une seule molécule de précurseur. C'est le cas en particulier pour les produits issus de l'anhydride pyromellitique comme indiqué plus loin.

Ces composés sont capables de libérer un alcool odorant de formule $R_1$ OH, lors de l'hydrolyse de la liaison ester. Par alcool odorant, on entend ici un alcool d'usage courant en parfumerie, c'est-à-dire utile à titre d'ingrédient parfumant pour la préparation de parfums ou d'articles parfumés. Bien que l'on ne puisse pas donner une liste exhaustive des alcools de formule $R_1$OH connus à ce jour qui peuvent être utilisés selon l'invention, on peut mentionner à titre d'exemple, l'alcool anisique, l'alcool cinnamique, l'alcool fenchylique, le 9-décén-1-ol, le phénéthylol, le citronellol (3,7-diméthyl-6-octén-1-ol), le 3-méthyl-5-phényl-1-pentanol (origine : Firmenich SA, Genève, Suisse), le Mayol® (7-p-menthan-1-ol ; origine : Firmenich SA, Genève, Suisse), le dihydromyrcénol (2,6-diméthyl-oct-7-én-2-ol), le alpha-ionol, le tétrahydro-ionol, le géraniol, le nérol, le (Z)-3-hexén-1-ol, le 1-hexanol, le 2-hexanol, le 3,3,5-triméthyl-hexanol, le 3,4,5,6,6-pentaméthyl-heptan-2-ol, le 5-éthyl-2-nonanol, le cis-6-nonénol, le 6,8-diméthyl-2-nonanol, le 2,6-nonadièn-1-ol, le bornéol, le 1-octèn-3-ol, le 4-cyclohexyl-2-méthyl-2-butanol (origine : Firmenich SA, Genève, Suisse), le 6-éthyl-3-méthyl-5-octèn-1-ol, le 3,7-diméthyl-oct-3,6-diénol, le 7-méthoxy-3,7-diméthyl-octan-2-ol, le 2-méthyl-1-phényl-2-propanol, le 1-phényléthanol, le 2-phényléthanol, le 2-phénylpropanol, le 3-phénylpropanol, le 2-méthyl-5-phénylpentanol, le 2-méthyl-4-phénylpentanol, le 3-méthyl-5-phénylpentanol, le cyclométhylcitronellol, le décanol, le dihydroeugénol, le 8-p-menthanol, le 3,7-diméthyl-1-octanol, le 2,6-diméthyl-2-heptanol, le dodécanol, l'octanol, l'undécanol, le 4-méthyl-3-décèn-1-ol, l'eugénol, le Florol® (tétrahydro-2-isobutyl-4-méthyl-4(2H)-pyranol ; origine : Firmenich SA, Genève, Suisse), le 2-phénoxy-éthanol, l'isoeugénol, le linalol, le Tarragol® (2-méthoxy-4-propyl-1-cyclohexanol ; origine: Firmenich SA, Genève, Suisse), la vanilline, l'éthyl-vanilline, le famésol, le cédrénol, le menthol, le p-menth-8-èn-3-ol, le 3,3,5-triméthyl-cyclohexanol, le 2,4,6-triméthyl-3-cyclohexényl-méthanol, le 4-(1-méthyléthyl)cyclohexyl-méthanol, le terpinéol, le tétrahydromuguol, le 3,7-diméthyl-3-octanol, le Polysantol® [(E)-3,3-diméthyl-5-(2',2',3'-triméthyl-3'-cyclopentèn-1-yl)-4-pentèn-2-ol ; origine : Firmenich SA. Genève, Suisse], le 2,2,6-triméthyl-alpha-propyl-cyclohexane propanol, le 5-(2,2,3-triméthyl-3-cyclopentyl)-3-méthylpentan-2-ol, le 3-méthyl-5-(2,2,3-trirnéthylcyclopentyl-3-ényl)pent-4-èn-2-ol, le 2-éthyl-4(2,2,3-triméthylcyclopentyl-3-ényl)but-2-èn-1-ol, le 4-(5,5,6-triméthylbicyclo[2.2.1]hept-2-yl)-cyclohexanol, le 2-(2-méthylpropyl)-4-hydroxy-4-méthyl-tétrahydropyrane, le 2-cyclohexyl propanol, le 2-(1,1-diméthyléthyl)-4-méthyl-cyclohexanol, le 1-(2-tert-butyl-cyclohexyloxy)-2-butanol, le 1-(4-isopropylcyclohexyl)-éthanol et le 1-(2,2,3,6-tétraméthyl-cyclohex-1-yl)-3-hexanol (origine: Firmenich SA, Genève, Suisse).

La particularité de l'invention tient dans le fait que l'hydrolyse qui provoque la libération de l'alcool est facilitée par un effet d'assistance du groupe nucléophile voisin de la fonction ester, le groupe CONH-$R_2$. Cet effet présente un avantage tout à fait inattendu à savoir qu'il permet le clivage de la liaison ester par une hydrolyse dans de simples conditions alkalines, comme le fait apparaître le schéma suivant :

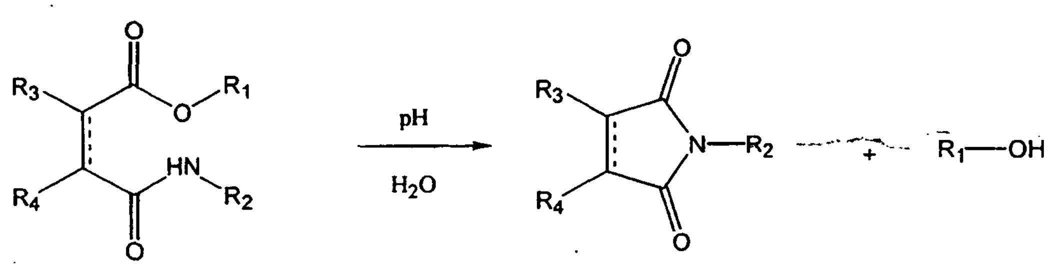


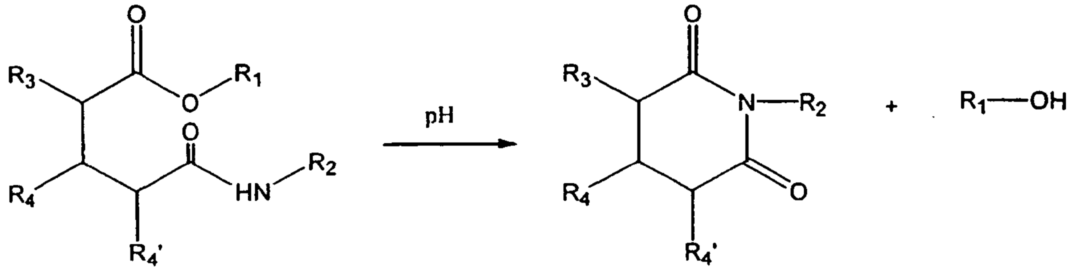

De telles conditions sont par exemple les conditions classiques d'un lavage de textile, au cours duquel s'effectue un changement de pH. Celui-ci passe en effet d'une valeur correspondant à un milieu acide vers des valeurs correspondant à un milieu neutre, voire basique au cours du cycle de lavage, permettant ainsi aux composés de l'invention de s'hydrolyser.

La réaction est par ailleurs naturellement catalysée en présence de chaleur. C'est le cas par exemple lors d'un séchage de linge notamment dans un séchoir électrique ou encore lors de son repassage, en particulier à la vapeur. La réaction d'hydrolyse conduit à la formation d'une molécule odorante $R_1OH$ dans laquelle $R_1$ a le sens indiqué plus haut, et d'un résidu du précurseur initial, une imide, ce résidu étant généralement inodore.

La réaction ne requiert aucun autre agent externe tel que par exemple la présence d'une lipase comme décrit dans l'art antérieur.

Ainsi, les composés de l'invention se révèlent être d'intéressants précurseurs d'alcools odorants. La réaction d'hydrolyse de la liaison ester dans les plus simples conditions comme cité plus haut, peut en outre être contrôlée du point de vue cinétique par le choix du substituant $R_2$. En effet, en fonction de la longueur de chaîne ou encore du degré de ramification de ce dernier, le relargage de l'alcool odorant s'effectuera de façon plus ou moins rapide. Ceci permet d'adapter le système selon l'invention aux besoins d'une application particulière et constitue par conséquent un avantage incontestable.

L'invention concerne également un procédé pour intensifier ou prolonger l'effet de diffusion de l'odeur caractéristique d'un alcool odorant développée par des textiles, caractérisé en ce qu'on soumet ces textiles à un cycle de lavage en présence d'un détergent et, facultativement à un traitement subséquent avec un adoucissant textile, ledit détergent et/ou adoucissant contenant un composé de l'invention.

Parmi les composés selon la formule (I) les plus appréciés pour la composition parfumante ou l'article parfumé sont les 2-carbamoylbenzoates de formule

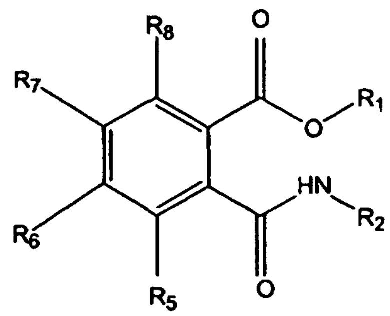

(Ia)

dans laquelle les symboles $R_5$, $R_6$, $R_7$ et $R_8$ pris indépendamment, représentent chacun un atome d'hydrogène ou un groupement alkyle linéaire ou ramifié de $C_1$ à $C_{20}$ et, lorsque pris deux à deux, peuvent former un ou plusieurs cycles.

On peut citer à titre préférentiel le 2-(octylcarbamoyl)benzoate de 3,7-diméthyl-6-octényle, le 2-(dodécylcarbamoyle)benzoate de 3,7-diméthyl-2,6-octadiényle et le 2-(dodécylcarbamoyl)benzoate de 3,7-diméthyl-6-octényle.

D'autre part, parmi les composés selon la formule (I) contenu en tant qu'ingrédients actif dans la composition ou l'article parfumé de la présente invention, les 3-carbamoylpropanoates et les 4-carbamoylbutanoates obéissant respectivement aux formules

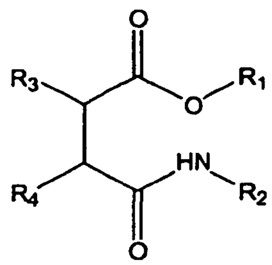

ou

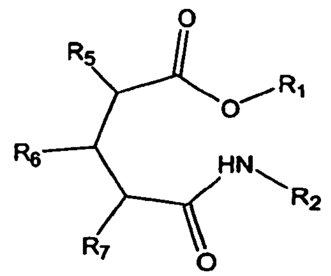

(Ib) (Ic)

dans lesquelles les symboles $R_3$, $R_4$, $R_5$, $R_6$ et $R_7$ représentent chacun un atome d'hydrogène ou un radical allcyle linéaire ou ramifié de $C_1$ à $C_{20}$ et pouvant contenir un ou plusieurs hétéroatomes, sont également appréciés.

On peut citer en particulier une préférence pour le 3-(octylcarbamoyl)propanoate de 3-phényl-2-propényle, le 4-(octylcarbamoyl)butanoate de 3,7-diméthyl-2,6-octadiényle et le 3-(octylcarbamoyl)propanoate de 3,7-diméthyl-2,6-octadiényle.

Par ailleurs, les composés présentant un double groupe fonctionnel ester-carbamoyle peuvent se révéler avantageux puisqu'ils sont capables de libérer deux molécules d'alcool odorant pour une seule molécule de précurseur. La présente invention concerne donc une composition parfumante ou un atricle parfumé contentant en tant qu'ingrédiant actif un composé de formule

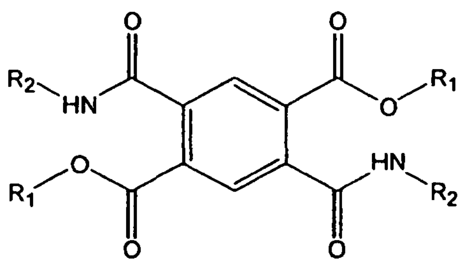

ou

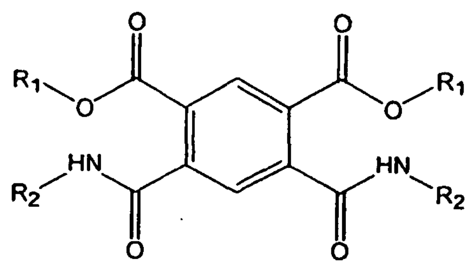

(Id)

dans lesquelles $R_1$ et $R_2$ ont le sens défini dans la formule (I). Parmi les composés (Id), on peut citer à titre préférentiel le 2,5-di(octylcarbamoyl)téréphtalate de di(3,7-diméthyl-2,6-octadiényl), le 1,3-di(octylcarbamoyl)isophtalate de di (3,7-diméthyl-2,6-octadiényl), ou un mélange de ces deux composés.

Les composés de l'invention peuvent être préparés à partir de composés commercialisés et à l'aide de méthodes classiques. Ainsi, d'une manière générale, à partir de produits de départ commerciaux (acides ou anhydrides) on crée une liaison ester par estérification classique des carboxyles, ou encore par catalyse acide. Puis, la fonction acide carboxylique restante est couplée à une amine primaire, respectivement à de l'ammoniac, pour donner la fonction carbamoyle mono-substituée, respectivement non substituée.

Par exemple, les 2-carbamoyle-benzoates (Ia) sont préparés à partir de l'anhydride phtalique (ou ses dérivés) suivant le schéma suivant :

## Schéma 1

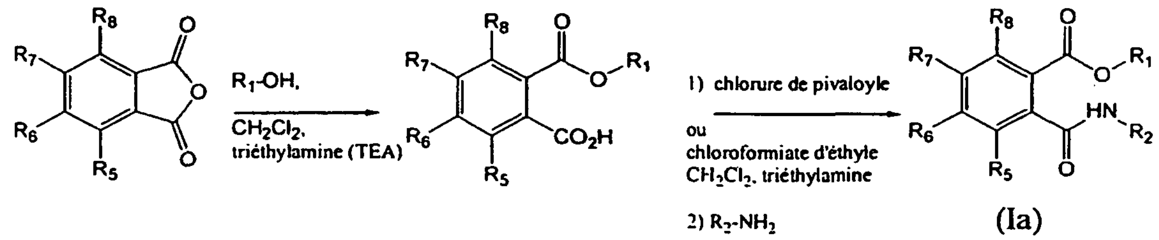


(Ia)

Les composés de formule (Ib) et (Ic) peuvent être synthétisés à partir d'anhydrides de type succinique ou glutarique, comme le montre le schéma suivant :

## Schéma 2

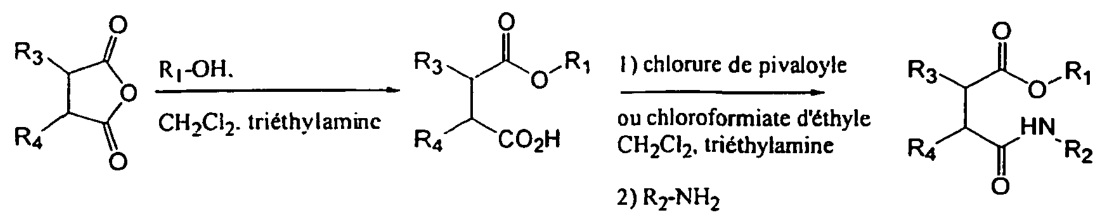


(Ib)

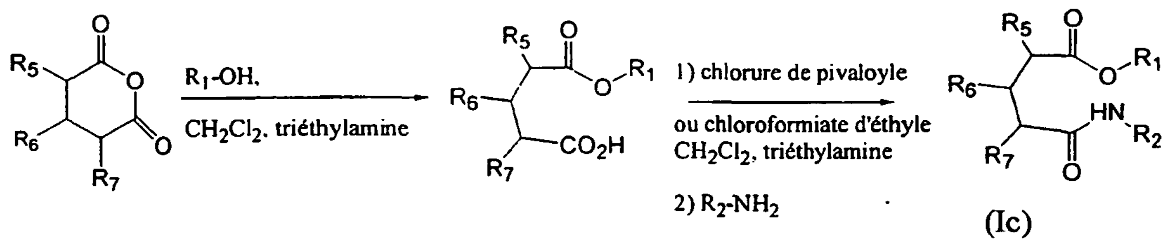


(Ic)

Enfin, les composés bifonctionnels de type (Id) sont préparés selon le même principe à partir de l'anhydride pyromellitique, comme le montre le schéma 3.

Schéma 3

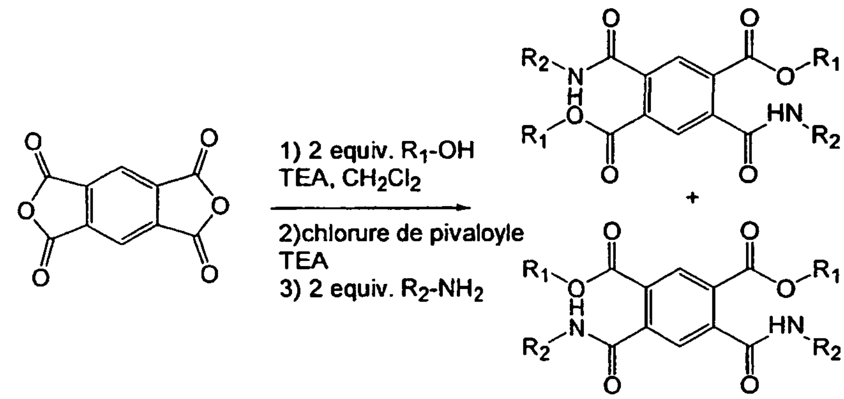


(Id)

Tous les symboles utilisés dans les schémas qui précèdent ont le sens indiqué dans la formule (I).

Les composés selon l'invention peuvent se prêter à toute application nécessitant l'effet de libération rapide ou prolongée d'un composant odorant tel que défini plus haut. Ils trouvent application en particulier en parfumerie fonctionnelle, notamment dans des applications telles que détergents liquides ou solides destinés au traitement de textiles et adoucissant textiles, pour lesquels on recherche des ingrédients dont l'odeur doit être impartie efficacement au textile lors du lavage. En effet, un des principaux avantages de l'invention réside dans le fait que les composés permettent d'impartir au linge une odeur intense, issue d'un alcool odorant qui ne serait pas senti suffisamment longtemps sur le linge si l'alcool avait été utilisé tel quel, c'est-à-dire sans précurseur.

On peut utiliser les composés de l'invention en tant qu'ingrédients parfumants pour le linge dans tous types de bases détergentes ou adoucissantes dans lesquelles ces composés sont stables. De préférence, on utilisera, comme c'est le cas général, des détergents à pH basique. Dans ce cas les composés devront être protégés d'une hydrolyse prématurée, par exemple par encapsulation. Quant aux adoucissants textiles, on préfère des produits dont le pH est inférieur à 7. A titre d'exemple, on peut utiliser des détergents du type de ceux décrits dans le brevet WO 97/34986. Par ailleurs on peut choisir comme bases adoucissantes celles décrites dans les brevets US 4,137,180, US 5,236,615, ou encore EP 799 885. D'autres compositions typiques de détergents et adoucissants pouvant être utilisés sont décrites dans des ouvragres tels que Ullman's Encyclopedia of Industrial Chemistry, Vol. A8, pages 315-448 (1987) et Vol. A25, pages 747-817 (1994); Flick, Advanced Cleaning Product Formulations, Noye Publication, Park Ridge, New Jersey (1989) ; Showell, in Surfactant Science Series, Vol 71 : Powdered Detergents, Marcel Dekher, New York (1998); Proceedings of the World Conference on Detergents (4$^{th}$, 1998, Montreux, Suisse), AOCS presse.

Bien entendu, l'utilisation des composés de l'invention n'est cependant pas limitée aux produits mentionnés auparavant. Ces composés se prêtent également à tous les autres emplois courants en parfumerie, à savoir au parfumage de savons et gels de douche ou de bain, de produits d'hygiène ou de traitement des cheveux comme les shampooings, ainsi que les déodorants corporels et désodorisants ambiants ou encore de préparations cosmétiques.

Les composés peuvent également être employés dans des applications telles que des compositions détergentes ou produits d'entretien destinés au nettoyage de la vaisselle ou de surfaces variées, qu'ils soient voués à un usage domestique ou industriel.

Dans ces applications, ils peuvent être utilisés seuls, en mélange entre eux, ou en mélange avec d'autres ingrédients parfumants, des solvants ou adjuvants d'usage courant en parfumerie. La nature et variété de ces coingrédients n'appelle pas une description plus détaillée ici, qui ne saurait d'ailleurs être exhaustive, l'homme de l'art étant à même de les choisir de par ses connaissances générales et en fonction de la nature du produit à parfumer et de l'effet olfactif recherché. Ces ingrédients parfumants appartiennent à des classes chimiques aussi variées que les alcools, aldéhydes, cétones, esters, éthers, acétates, nitriles, hydrocarbures terpéniques, composés hétérocycliques azotés ou soufrés, ainsi que des huiles essentielles d'origine naturelle ou synthétique. Beaucoup de ces ingrédients sont d'ailleurs répertoriés dans des textes de référence tels que le livre de S. Arctander, Perfume and Flavor Chemicals, 1969, Montclair, New Jersey, USA, ou ses versions plus récentes, ou dans d'autres oeuvres de nature similaire.

Les proportions dans lesquelles les composés selon l'invention peuvent être incorporés dans les divers produits susmentionnés varient dans une gamme de valeurs étendue. Ces valeurs sont dépendantes de la nature de l'article ou produit que l'on veut parfumer et de l'effet olfactif recherché, ainsi que de la nature des coingrédients dans

une composition donnée lorsque les composés de l'invention sont utilisés en mélange avec des coingrédients parfumants, des solvants ou des adjuvants d'usage courant dans l' art.

A titre d'exemple, on peut citer des concentrations typiques de l'ordre de 0,1 à 5%, voire plus, en poids de ces composés par rapport au poids de la composition dans laquelle ils sont incorporés. Des concentrations inférieures à celles-ci peuvent être utilisées lorsque ces composés sont directement appliqués dans le parfumage des produits de consommation divers cités auparavant.

L'invention sera maintenant décrite de façon plus détaillée dans les exemples suivants dans lesquels les températures sont indiquées en degrés Celsius et les abréviations ont le sens usuel dans l'art.

Exemple 1

Préparation des composés de formule (I)

Les composés de l'invention ont tous été préparés selon la même méthode générale. Pour chaque composé, la nature de l'anhydride d'acide utilisé, l'alcool odorant, le solvant (MTBE : méthyl *tert*-butyl éther, ou $CH_2Cl_2$: dichlorométhane), le type d'anhydride mixte utilisé, généré par le chlorure de pivaloyle ou le chloroformiate d'éthyle, et enfin l'amine choisie (acétate d'ammonium, éthylamine, octylamine ou dodecylamine, etc...) sont précisés.

**Méthode générale de synthèse**

De l'anhydride d'acide (1 équivalent) et un alcool parfumant (1 équivalent, respectivement 2 équivalents si l'anhydride est bifonctionnel, comme c'est le cas par exemple de l'anhydride pyromellitique) ont été mis en solution dans un solvant (20 ml/mmol d'anhydride). Le mélange a été refroidi à une température de 5 à 10°C avant d'ajouter de la triéthylamine (1 équivalent, respectivement 2 équivalents dans le cas de l'anhydride pyromellitique). Le mélange a été maintenu sous agitation de 2 à 4 h à température ambiante. Le milieu réactionnel a alors été refroidi à 0°C, un équivalent de triethylamine (2 équivalents pour les composés bifonctionnels) a été ajouté, puis goutte à goutte 1 équivalent (ou 2 équivalents pour les composés bifonctionnels) de chloroformiate d'éthyle ou de chlorure de pivaloyle pour former l'anhydride mixte désiré. On a laissé réagir pendant 1 h à température ambiante avant d'ajouter 1 équivalent (ou 2 équivalents pour les composés bifonctionnels) de l'amine choisie. On a laissé réagir encore entre 2 et 4 h à température ambiante. On a ajouté 5 volumes d'acétate d'éthyle, lavé à l'aide de $KHSO_4$ à 5%, puis à la saumure. On a séché sur sulfate de sodium, puis évaporé les solvants. Les produits ont été utilisés soit tels quels, soit purifiés par cristallisation ou par chromatographie comme il est précisé pour chaque produit.

*1. 2-carbamoylbenzoate de (Z)-3-hexényle*

La synthèse a été effectuée suivant la méthode générale décrite plus haut, à partir de l'anhydride phtalique et du (Z)-3-hexénol. L'anhydride mixte a été formé. à l'aide de chloroformiate d'éthyle. L'amine choisie était l'acétate d'ammonium (sur 11.25 mmol) et le solvant MTBE, TEA. On a obtenu un rendement brut de 90%, et 50% après recristallisation dans l'éther.
Données analytiques :

$^1$H RMN($CDCl_3$, $\delta$, ppm) : 7,84(d, 1H) ; 7,54-7,43(m, 3H); 6,26(d large, 2H) ; 5,52(m, 1H) ; 5,38(m, 1H) ; 4,28(t, J=6,7Hz, 2H); 2,49(dd, J=7,1, 6,7Hz, 2H) ; 2,07(m, J=7,5, 7.1Hz, 2H) ; 0,96(t, J=7,5Hz, 3H).
$^{13}$C RMN($CDCl_3$, $\delta$, ppm) : 171,5(s), 166,9(s), 137,4(s), 134,7(d), 131,8(d), 129,9(d), 129,8(d), 129,5(s), 127,6(d), 123,6(d), 65,2(t), 26,6(t), 20,7(t), 14,2(q).
SM (CI, $NH_3$) : 248,1(10, $M+H^+$), 165,9(100).

*2. 2-(éthylcarbamoyl)benzoate de (Z)-3-hexényle*

La synthèse a été effectuée suivant la méthode générale décrite plus haut, à partir de l'anhydride phtalique et du (Z)-3-hexénol. L'anhydride mixte a été formé à l'aide de chloroformiate d'éthyle. L'amine choisie était le chlorhydrate d'ethylamine (sur 11,25 mmol), et le solvant MTBE, TEA. On a obtenu un rendement de 30% après chromatographie (50/50 Cyclohexane/Acétate d'éthyle).
Données analytiques:

$^1$H RMN($CDCl_3$, $\delta$, ppm) : 7,84(d, 1H) ; 7,53-7,42(m, 3H) ; 5,96(t large, 1H) ; 5,52(m, 1H) ; 5,37(m, 1H) ; 4,27(t, J=7,1Hz, 2H) ; 3,47(dq, J=7,1, 5,6Hz, 2H); 2,48(dd, J=7,1, 6,8Hz, 2H); 2,07(m, J=7,4, 6,8Hz, 2H); 1,24(t, J=7,1Hz, 3H) ; 0,96(t, J=7,4 Hz, 3H).

$^{13}C$ RMN($CDCl_3$, δ, ppm): 169,3(s), 166,8(s), 138,4(s), 134,7(d), 131,8(d), 130,0(d), 129,5(d), 129,4(s), 127,7(d), 123,5(d), 65,0(t), 35,0(t), 26,6(t), 20,6(t), 14,7(q), 14,2(q).
SM (CI, $NH_3$): 276,1(18, $M+H^+$), 194,0(100), 176,0(95), 159,9(25).

*3. 2-(dodécylcarbamoyl)benzoate de (Z)-3-hexényle*

La synthèse a été effectuée suivant la méthode générale décrite plus haut, à partir de l'anhydride phtalique et du (Z)-3-hexénol. L'anhydride mixte a été formé à l'aide de chlorure de pivaloyle. L'amine choisie était la dodécylamine (sur 11.25 mmol) et le solvant MTBE. La dernière étape a été effectuée dans le dichlorométhane et TEA. On a obtenu un rendement de 89 %.

$^1H$. RMN($CDCl_3$. δ, ppm): 7,84(m. 1H) ; 7,53-7.42(m. 3H); 5.95(t large, J=5,4Hz, 1H) ; 5.52(m, 1H) ; 5,37(m, 1H) ; 4,27(t, J=7,1Hz, 2H) ; 3.42(dt, J=7,1, 5,4Hz, 2H); 2,48(~dt, J=7,1 Hz, 2H); 2,07(~dq, J=7,5Hz. 2H); 1.61(m, 2H); 1,39-1,21(m, 18H); 0,96(t, J=7,5Hz, 3H); 0,88(t, J=6,8Hz, 3H).
$^{13}C$ RMN($CDCl_3$, δ, ppm) : 169,2(s), 166,8(s), 138,5(s), 134,7(d), 131,8(d), 130,0(d), 129,4(d), 127,7(d), 123,5(d), 65,0(t), 40,2(t), 31,9(t), 29,6(t), 29,5(t), 29,4(t), 27,0(t), 26,7(t), 20,7(t), 14,2(q), 14,1(q).
SM (ESI) : 416,3(100, $M+H^+$).

*4. 2-carbamoylbenzoate de (E)-3, 7-diméthyl-2, 6-octadiényle*

La synthèse a été effectuée suivant la méthode générale décrite plus haut, à partir de l'anhydride phtalique et du 3,7-diméthyl-2,6-octadiénol. L'anhydride mixte a été formé à l'aide de chloroformiate d'éthyle. L'amine choisie était l'acétate d'ammonium (sur 10 mmol) et le solvant MTBE, TEA. On a obtenu un rendement de 43% après chromatographie rapide (50/50 cyclohexane/acétate d'éthyle)
Données analytiques :

$^1H$ RMN($CDCl_3$, δ, ppm) : 7,86(d, J=7,2Hz, 1H) ; 7,54-7,44(m, 3H) ; 6,08(s large, 2H) ; 5,45(m, 1H) ; 5,09(m, 1H) ; 4,83(d, J=7,0Hz, 2H) ; 2,16-2,05(m, 4H) ; 1,75(s, 3H) ; 1,67(s, 3H) ; 1,60(s, 3H).
$^{13}C$ RMN($CDCl_3$, δ, ppm) : 171,4(s), 166,9(s), 142,9(s), 137,2(s), 131,9(s), 131,8(d), 130,0(d), 129,9(d), 129,7(s), 127,6(d), 123,7(d), 117,9(d), 62,6(t), 39,6(t), 26,3(t), 25,7(q), 17,7(q), 16,6(q).
SM (CI, $NH_3$) : 319(6, $M+NH4^+$), 302(5, $M+H^+$), 183(100), 166(57).

*5. 2-(octylcarbamoyl)benzoate de (E)-3,7-diméthyl-2,6-octadiényle*

La synthèse a été effectuée suivant la méthode générale décrite plus haut, à partir de l'anhydride phtalique et du 3,7-diméthyl-2,6-octadiénol. L'anhydride mixte a été formé à l'aide de chloroformiate d'éthyle. L'amine choisie était l'octylamine (sur 10 mmol) et le solvant MTBE, TEA. On a obtenu un rendement de 75% après chromatographie (70/30 cyclohexane/acétate d'éthyle).
Données analytiques :

$^1H$ RMN($CDCl_3$, δ, ppm) : 7,87(m, 1H) ; 7,53-7,42(m, 3H) ; 5.90(t. J=5,4Hz, 1H) ; 5,43(dt, J=7.1, 0.8Hz, 1H) ; 5,09 (m, 1H) : 4.81 (d, J=7.3Hz, 2H) ; 3,42(dt, J=7,1, 5,4Hz, 2H); 2,15-2,03 (m, 4H); 1,74(s, 3H); 1,68(s, 3H); 1,64-1,56 (m, 2H) ; 1,60(s, 3H) ; 1,42-1,21(m, 10H) ; 0,88(t, J=6,9Hz, 3H).
$^{13}C$ RMN($CDCl_3$, δ, ppm) : 169,3(s), 166,8(s), 142,5(s), 138,5(s), 131,9(s), 131,8(d), 130,1(d), 129,4(d), 127,7(d), 123,7(d), 118,0(d), 62,5(t), 40,2(t), 39,6(t), 31,8(t), 29,5(t), 29,32(t), 29,26(t), 27,1(t), 26,3(t), 25,7(q), 22,7(t), 17,7 (q), 16,6(q), 14,1(q).
SM (CI, $NH_3$) : 414(12, $M+H^+$), 278(100), 260(82).

*6. 2-(dodécylcarbamoyl)benzoate de (E)-3,7-diméthyl-2,6-octadiényle*

La synthèse a été effectuée suivant la méthode générale décrite plus haut, à partir de l'anhydride phtalique et du 3,7-diméthyl-2,6-octadiénol. L'anhydride mixte a été formé à l'aide de chloroformiate d'éthyle. L'amine choisie était la dodécylamine (sur 10 mmol) et le solvant MTBE, TEA. On a obtenu un rendement de 68% après chromatographie (80/20 cyclohexane/acétate d'éthyle).
Données analytiques :

$^1H$ RMN($CDCl_3$, δ, ppm) : 7,86(m, 1H) ; 7,52-7,42(m, 3H) ; 5,92(t, J=5,4Hz, 1H) ; 5,43(~t, J=7,1 Hz, 1H) ; 5,09(m, 1H) ; 4,81 (d, J=7,1 Hz, 2H) ; 3,41 (dt, J=7,1, 5,4Hz, 2H); 2,15-2,03(m, 4H); 1,73(s, 3H); 1,68(s, 3H); 1,64-1,56(m,

2H) ; 1,60(s, 3H) ; 1,39-1,20(m, 18H) ; 0,88(t, J=6,8Hz, 3H).
$^{13}C$ RMN($CDCl_3$, δ, ppm) : 169,3(s), 166,8(s), 142,5(s), 138,5(s), 131,84(s), 131,77(d), 130,1(d), 129,5(d), 129,4 (s), 127,7(d), 123,7(d), 118,1(d), 62,5(t), 40,2(t), 39,6(t), 31,9(t), 29,7(t), 29,6(t), 29,5(t), 29,4(t), 27,1(t), 26,3(t), 25,7(q), 22,7(t), 17,7(q), 16,6(q), 14,1(q).
SM (ESI) : 470(100, $M+H^+$), 334(80).

*7. 2-(isopropylcarbamoyl)benzoate de (E)-3,7-diméthyl-2,6-octadiényle*

La synthèse a été effectuée suivant la méthode générale décrite plus haut, à partir de l'anhydride phtalique et du 3,7-diméthyl-2,6-octadiénol. L'anhydride mixte a été formé à l'aide de chloroformiate d'éthyle. L'amine choisie était l'isopropylamine (sur 5,3 mmol) et le solvant dichlorométhane, TEA. On a obtenu un rendement de 64% après chromatographie (70/30 cyclohexane/acétate d'éthyle).
Données analytiques :

$^1H$ RMN($CDCl_3$, δ, ppm): 7,86(m, 1H) ; 7,53-7,42(m, 3H); 5,72(d, J=7.5Hz, 1H) ; 5,43(m, 1H) ; 5.09(m, 1H) ; 4,82 (d, J=7,1 Hz, 2H); 4,26(m, 1H) ; 2,14-2,01(m, 4H) ; 1,74(s, 3H) ; 1,68(s, 3H) ; 1,60(s, 3H) ; 1,26(d, J=6,7Hz, 6H).
$^{13}C$ RMN($CDCl_3$, δ, ppm) : 168,5(s), 166,8(s), 142,4(s), 138,5(s), 131,9(s), 131,7(d), 130,1(d), 129,5(s), 129,4(d), 127,6(d), 123,7(d), 118,1(d), 62,5(t), 42,0(d), 39,6(t), 26,3(t), 25,7(q), 22,7(q), 17,7(q), 16,6(q).
SM (CI, $NH_3$) : 344(35, $M+H^+$), 225(80), 208(100), 154(10).

*8. 2-[(1-méthylpropyl)carbamoyl]benzoate de (E)-3,7-diméthyl-2, 6-octadiényle*

La synthèse a été effectuée suivant la méthode générale décrite plus haut, à partir de l'anhydride phtalique et du 3,7-diméthyl-2,6-octadiénol. L'anhydride mixte a été formé à l'aide de chloroformiate d'éthyle. L'amine choisie était l'isopropylamine (sur 5,3 mmol) et le solvant dichlorométhane, TEA. On a obtenu un rendement de 83% après chromatographie (80/20 cyclohexane/acétate d'éthyle).
Données analytiques :

$^1H$ RMN($CDCl_3$, δ, ppm): 7,85(m, 1H) ; 7,53-7,42(m, 3H); 5,68(d, J=7,9Hz, 1H) ; 5,43(m, 1H) ; 5,09(m, 1H) ; 4,82 (d, J=7,1 Hz, 2H); 4,10(m, 1H) ; 2,14-2,02(m, 4H); 1,74(s, 3H) ; 1,68(s, 3H); 1,62-1,48(m, 2H); 1,60(s, 3H) ; 1,23 (d, J=6,3Hz, 3H) ; 0,99(t, J=7,3Hz, 3H).
$^{13}C$ RMN($CDCl_3$, δ, ppm) : 168,6(s), 166,9(s), 142,4(s), 138,6(s), 131,8(s), 131,7(d), 130,1(d), 129,6(s), 129,4(d), 127,7(d), 123,8(d), 118,1 (d), 62,5(t), 47,2(d), 39,6(t), 29,6(t), 26,9(t), 25,7(q), 20,2(q), 17,7(q), 16,6(q), 10,4(q).
SM (CI, $NH_3$) : 358(45, $M+H^+$), 239(50), 222(100), 154(15).

*9. 2-(dodécylcarbamoyl)benzoate de (E)-3-phényl-2-propényle*

La synthèse a été effectuée suivant la méthode générale décrite plus haut, à partir de l'anhydride phtalique et du 3-phényl-2-propénol. L'anhydride mixte a été formé à l'aide de chlorure de pivaloyle. L'amine choisie était la dodécylamine (sur 10 mmol) et le solvant MTBE, TEA. On a obtenu un rendement brut de 93 %, et 30 % après cristallisation.
Données analytiques :

$^1H$ RMN($CDCl_3$, δ, ppm): 7,89(m, 1H) ; 7,53-7,23(m, 8H) ; 6,71(d élargi, J=15,8Hz, 1H) ; 6,36(dt, J=15,8, 6,3Hz, 1H) ; 5,90(t, J=5,3Hz, 1H) ; 4,93(dd, J=6,3, 1,2Hz, 2H); 3,38(dd, J=7,2, 5,3Hz, 2H); 1,55(m, 2H); 1,30-1,20(m, 18H) ; 0,88(t, J=7,2Hz, 3H).
$^{13}C$ RMN($CDCl_3$, δ, ppm) : 169,3(s), 166,6(s), 138,6(s), 136,2(s), 134,6(d), 132,0(d), 130,1 (d), 129,5(d), 129,2 (s), 128,6(d), 128,1(d), 127,6(d), 126,7(d), 122,8(d), 66,1(t), 40,3(t), 31,9(t), 29,7(t), 29,6(t), 29,4(t), 29,43(t), 29,37 (t), 27,0(t), 22,7(t), 14,1(q).
SM (CI, $NH_3$) : 467(52, $M+NH_4^+$), 450(32, $M+H^+$), 351(100), 151(68).

*10. 3-(octylcarbamoyl)propanoate de (E)-3-phényl-2-propényle*

La synthèse a été effectuée suivant la méthode générale décrite plus haut, à partir de l'anhydride succinique et du 3-phényl-2-propénol. L'anhydride mixte a été formé à l'aide de chlorure de pivaloyle. L'amine choisie était l'octylamine (sur 100 mmol) et le solvant dichlorométhane, TEA. On a obtenu un rendement de 58% après recristallisation dans l'éther.
Données analytiques :

$^1H$ RMN($CDCl_3$, δ, ppm): 7,39-7,23(m, 5H); 6,64(d large, J=16,2Hz. 1H) ; 6,26(dt, J=16,2, 6,4Hz, 1H) ; 5,83(~t, 1H) ; 4,74(dd, J=6,4, 1,2Hz, 2H) ; 3,22(m, 2H) ; 2,72(t, J=6,8Hz, 2H) ; 2,48(t, J=6,8Hz, 2H) ; 1,47(m, 2H) ; 1,31-1,20 (m, 10H) ; 0,87(t, J=6,7Hz, 3H).
$^{13}C$ RMN($CDCl_3$, δ, ppm) : 172,9(s), 171,2(s), 136,2(s), 134,3(d), 128,6(d), 128,1(d), 126,6(d), 123,0(d), 65,3(t), 39,7(t), 31,8(t), 31,1(t), 29,7(t), 29,6(t). 29,3(t), 29,2(t), 26,9(t), 22,6(t), 14,1(q).
SM (ESI) : 346,1(100, M+H$^+$).

*11. 2-carbamoylbenzoale de (R)-3, 7-diméthyl-6-octényle*

La synthèse a été effectuée suivant la méthode générale décrite plus haut, à partir de l'anhydride phtalique et du 3,7-diméthyl-6-octénol. L'anhydride mixte a été formé à l'aide du chloroformiate d'éthyle. L'amine choisie était l'acétate d'ammonium (sur 10 mmol) et le solvant MTBE, TEA. On a obtenu un rendement de 72% après chromatographie rapide (50/50 cyclohexane/acétate d'éthyle).
Données analytiques :

$^1H$ RMN($CDCl_3$, δ, ppm) : 7,85(d, J=7,5Hz, 1H) ; 7,55-7,45(m, 3H); 6,16(d large, 2H) ; 5,09(m, 1H) ; 4,34(m, 2H) ; 2,00(m, 2H) ; 1,79(m, 1H) ; 1,67(s, 3H); 1,65-1,50(m, 1H) ; 1,60(s, 3H); 1,39(m, 1H) ; 1,23(m, 2H); 0,95(t, J=6,3Hz, 3H).
$^{13}C$ RMN($CDCl_3$, δ, ppm): 171,5(s), 166,9(s), 137,3(s), 131,8(d), 131,4(s), 129,92(d), 129,85(d), 129,6(s), 127,6 (d), 124,6(d), 64,3(t), 37,0(t), 35,3(t), 29,5(d), 25,7(q), 19,4(q), 17,7(q).
SM (CI, $NH_3$) : 304(100, M+H$^+$), 183(10), 166(18), 148(20).

*12. 2-(octylcarbamoyl)benzeate de (R)-3, 7-diméthyl-6-octényle*

La synthèse a été effectuée suivant la méthode générale décrite plus haut, à partir de l'anhydride phtalique et du 3,7-diméthyl-6-octénol. L'anhydride mixte a été formé à l'aide de chlorure de pivaloyle. L'amine choisie était l'octylamine (sur 10 mmol) et le solvant MTBE, TEA. On a obtenu un rendement de 47% après chromatographie (80/20 cyclohexane/acétate d'éthyle).
Données analytiques :

$^1H$ RMN($CDCl_3$, δ, ppm): 7,85(m, 1H) ; 7,54-7,43(m, 3H) ; 5,91(t, J=5,3Hz, 1H) ; 5,09(m, 1H) ; 4,32(m, 2H); 3,43 (~dd, J=7,3, 5,3Hz, 2H); 2,08-1,91(m, 2H); 1,94-1,73(m, 1H) ; 1,67(s, 3H); 1,64-1,49(m, 3H); 1,60(s, 3H) ; 1,43-1,20 (m, 13H) ; 0,95(d, J=6,3Hz, 3H) ; 0,88(t, J= 6,7Hz, 3H).
$^{13}C$ RMN($CDCl_3$, δ, ppm) : 169,3(s), 166,9(s), 138,5(s), 131,8(d), 131,4(s), 130,0(d), 129,5(d), 127,7(d), 124,6(d), 64,2(t), 40,2(t), 37,0(t), 35,4(t), 31,8(t), 29,53(d), 29,48(t), 29,32(t), 29,25(t), 27,0(t), 25,7(q), 25,4(t), 22,7(t), 19,4 (q), 17,7(q), 14,1(q).
SM (ESI) : 416,3(100, M+H$^+$).

*13. 2-(dodécylcarbamoyl)benzoate de (R)-3.7-diméthyl-6-octényle*

La synthèse a été effectuée suivant la méthode générale décrite plus haut, à partir de l'anhydride phtalique et du 3.7-diméthyl-6-octénol. L'anhydride mixte a été formé à l'aide de chlorure de pivaloyle. L'amine choisie était l'octylamine (sur 10 mmol) et le solvant MTBE, TEA. On a obtenu un rendement de 67% après chromatographie (80/20 cyclohexane/acétate d'éthyle).
Données analytiques :

$^1H$ RMN($CDCl_3$, δ, ppm) : 7,84(m, 1H) ; 7,53-7,42(m, 3H) ; 5,95(t, J=5,3Hz, 1H) ; 5,09(m, 1H) ; 4,32(m, 2H) ; 3,42 (dd, J=7,1, 5,3Hz, 2H) ; 2,08-1,91(m, 2H) ; 1,85-1,73(m, 1H) ; 1,67(s, 3H) ; 1,64-1,49(m, 3H) ; 1,60(s, 3H) ; 1,43-1,20(m, 21H); 0,95(d, J=6,3Hz, 3H) ; 0,88(t, J=7,1Hz, 3H).
$^{13}C$ RMN($CDCl_3$, δ, ppm) : 169,3(s), 166,9(s), 138,5(s), 131,8(d), 131,4(s), 130,0(d), 129,5(s), 129,4(d), 127,7(d), 124,6(d), 64,2(t), 40,2(t), 37,0(t), 35,4(t), 31,9(t), 29,7(t), 29,6(t), 29,52(d), 29,48(t), 29,4(t), 27,6(t), 27,1(t), 27,0 (t), 25,7(q), 25,4(t), 22,7(t), 19,4(q), 17,7(q), 14,1(q).
SM (ESI) : 472,3(100, M+H$^+$).

*14. 2-carbamoylbenzoate de (4-isopropyl-1-cyclohexyl)methyle*

La synthèse a été effectuée suivant la méthode générale décrite plus haut, à partir de l'anhydride phtalique et du Mayol® [4-(1-méthyléthyl)cyclohexaneméthanol ; origine : Firmenich SA, Genève, Suisse]. L'anhydride mixte a

été formé à l'aide de chloroformiate d'éthyle. L'amine choisie était l'acétate d'ammonium (sur 10 mmol) et le solvant MTBE, TEA. On a obtenu un rendement de 40% après chromatographie rapide (50/50 cyclohexane/acétate d'éthyle). Données analytiques :

$^1$H RMN($CDCl_3$, δ, ppm) : 7,85(d, J=7,1Hz, 1H) ; 7,55-7,45(m, 3H) ; 6,26(d large, 2H) ; 4,25(d, J=7,6Hz, 2H, isomère *cis* 67%) ; 4,10(d, J=6,3Hz, 2H, isomère *trans* 33%); 2,04(m, 1H) ; 1,87(m, 1H) ; 1,77(m, 1H) ; 1,64-1,44(m, 4H); 1,41-1,30(m, 2H) ; 1,15-1,07(m, 1H) ; 1,01(m, 1H) ; 0,86(d, J=6,7Hz, 6H, isomère *cis*) ; 0,85(d, J=6,6Hz, 6H, isomère *trans*).

$^{13}$C RMN($CDCl_3$, δ, ppm): 171,6(s), 167,1(s), 137,3(s), 131,8(d), 130,0(d), 129,8(d). 129,7(s), 71 (t, isomère *trans),* 68,1 (t, isomère *cis),* 43,9(d, *trans*), 42.9(d. *cis*). 37,3(d). 33,7(d), 32,8(d), 30,5(d). 29,9(t), 29,0(t), 26,4(t). 25,5(t). 20,3(q, *cis),* 19,8(q, *trans*).

SM (CI, $NH_3$) : 304(40, M+H$^+$). 166(95), 148(100).

*15. 2(octylcarbamoyl)benzoate de (4-isopropyl-1-cyclohexyl)méthyle*

La synthèse a été effectuée suivant la méthode générale décrite plus haut, à partir de l'anhydride phtalique et du Mayol®. L'anhydride mixte a été formé à l'aide de chlorure de pivaloyle. L'amine choisie était l'octylamine (sur 10 mmol) et le solvant MTBE, TEA. On a obtenu un rendement de 53% après chromatographie (85/15 cyclohexane/acétate d'éthyle).
Données analytiques :

$^1$H RMN($CDCl_3$, δ, ppm): 7,86(d, J=7,1 Hz, 1H) ; 7,53-7,42(m, 3H) ; 5,97(t, J=5,3Hz, 1H) ; 4,23(d, J=7,5Hz, 2H, isomère *cis* 68%) ; 4,09(d, J=6,7Hz, 2H, isomère *trans* 32%) ; 3,41(dd, J=7,1, 5,3Hz, 2H) ; 2,06-1,96(m, 1H) ; 1,85 (m, 1H) ; 1,76(m, 1H) ; 1,64-1,42(m, 4H); 1,40-1,20(m, 14H); 1,15-1,06(m, 1H) ; 1,01(m, 1H) ; 0,90-0,85(m, 9H).

$^{13}$C RMN($CDCl_3$, δ, ppm) : 169,2(s), 167,0(s), 138,5(s), 131,8(d), 130,0(d), 129,5(s), 129,4(d), 127,7(d), 70,8(t, isomère *trans),* 68,0(t, isomère *cis*), 43,9(d, *trans),* 42,9(d, *cis),* 40,2(t), 37,3(d), 33,7(d), 32,8(d), 31,8(t), 30,5(d), 29,9(t), 29,5(t), 29,32(t), 29,25(t), 29,0(t), 27,1(t), 26,4(t), 25,5(t), 22,7(t), 20,3(q, *cis),* 19,8(q, *trans),* 14,1(q).

SM (ESI) : 416,3(100, M+H).

*16. 2-(dodécylcarbamoyl)benzoate de (4-isopropyl-1-cyclohexyl)méthyle*

La synthèse a été effectuée suivant la méthode générale décrite plus haut, à partir de l'anhydride phtalique et du Mayol®. L'anhydride mixte a été formé à l'aide de chlorure de pivaloyle. L'amine choisie était la dodécylamine (sur 10 mmol) et le solvant MTBE, TEA. On a obtenu un rendement de 60% après chromatographie (85/15 cyclohexane/acétate d'éthyle).
Données analytiques :

$^1$H RMN($CDCl_3$, δ, ppm): 7,86(m, 1H) ; 7,53-7,42(m, 3H); 5,92(t, J=5,2Hz, 1 H) ; 4,24(d, J=7,Hz, 2H, isomère *cis* 69%) ; 4,09(d, J=6,7Hz, 2H, isomère *trans* 31%) ; 3,41 (dd, J=7,1, 5.2Hz. 2H) ; 2,02(m, 1H) ; 1.86(m. 1H) ; 1.75 (m, 1H) ; 1 ,64- 1 ,43(m, 4H); 1.40-1,20(m, 22H); 1 ,15-1,06(m, 1H) ; 1.01(m, 1H) ; 0.90-0,85(m. 9H).

$^{13}$C RMN($CDCl_3$, δ, ppm) : 169,3(s), 167,0(s), 138,5(s), 131,8(d), 130,0(d), 129,5(s), 129,4(d), 127,7(d), 70,8(t, isomère *trans),* 68,0(t, isomère *cis*), 43,9(d, *trans),* 42,9(d, *cis),* 40,2(t), 37,3(d), 33,7(d), 32,9(d), 31,9(t), 30,5(d), 29,9(t), 29,7(t), 29,6(t), 29,5(t), 29,4(t), 29,0(t), 27,0(t), 26,4(t), 25,5(t), 22,7(t), 20,3(q, *cis),* 19,8(q, *trans),* 14,1(q).

SM (ESI) : 472,5(100, M+H$^+$).

*17. 2-(octylcarbamoyl)benzoate de 2-phényléthyle*

La synthèse a été effectuée suivant la méthode générale décrite plus haut, à partir de l'anhydride phtalique et du phényléthanol. L'anhydride mixte a été formé à l'aide du chlorure de pivaloyle. L'amine choisie était l'octylamine (sur 10 mmol) et le solvant MTBE, TEA. On a obtenu un rendement de 40% après chromatographie (80/20 cyclohexane/acétate d'éthyle).
Données analytiques :

$^1$H RMN($CDCl_3$, δ, ppm) : 7,80(m, 1H) ; 7,53-7,41(m, 3H) ; 7,33-7,21(m, 5H) ; 5,85(t élargi, J=5,3Hz, 1H) ; 4,50(t, J=7,1Hz, 2H) ; 3,39(~dd, J=7,1, 5,3Hz, 2H); 3,05(t, J=7,1Hz, 2H); 1,59(m, 2H) ; 1,40-1,21(m, 10H); 0,88(t, J=7,1 Hz, 3H).

$^{13}$C RMN($CDCl_3$, δ, ppm) : 169,2(s), 166,6(s), 138,6(s), 137,5(s), 131,9(d), 130,0(d), 129,5(d), 129,3(s), 128,9(d), 128,6(d), 127,7(d), 126,6(d), 66,0(t), 40,2(t), 35,0(t), 31,8(t), 29,5(t), 29,3(t), 29,2(t), 27,1(t), 22,6(t), 14,1(q).

SM (CI, $NH_3$) : 399(7, $M+NH_4^+$), 382(85, $M+H^+$), 354(12), 277(100), 261(20), 180(20).

*18. 2-(dodécylcarbamoyl)benzoate de 2-phényléthyle*

La synthèse a été effectuée suivant la méthode générale décrite plus haut, à partir de l'anhydride phtalique et du phényléthanol. L'anhydride mixte a été formé à l'aide du chlorure de pivaloyle. L'amine choisie était la dodécylamine (sur 10 mmol) et le solvant MTBE, TEA. On a obtenu un rendement de 92%.
Données analytiques :

$^1H$ RMN($CDCl_3$, $\delta$, ppm): 7,80(d, J=7,8Hz, 1H) ; 7.54-7,41(m, .3H) ; 7.33-7.21 (m, 5H) ; 5.91 (t. J=5,2Hz. 1H) ; -, 4.49(t, J=7.2Hz. 2H) ; 3,39(~dd. J=7.1. 5,2Hz, 2H); 3,04(t, J=7,1Hz, 2H); 1,58(m, 2H); 1,33-1.23(m, 18H) ; 0,88(t, J=7,1Hz, 3H).
$^{13}C$ RMN($CDCl_3$, $\delta$, ppm) : 169,4(s), 166,7(s), 138,5(s), 137,6(s), 131,9(d), 130,0(d), 129, 5(d), 129,2(s), 128,9 (d), 128,6(d), 127,7(d), 126,6(d), 66,0(t), 40,2(t), 35,0(t), 31,9(t), 29,6(t), 29,4(t), 29, 3(t), 22,7(t), 14,1(q).
SM (CI, $NH_3$) : 455(32, $M+NH_4^+$), 438(45, $M+H^+$), 333(100), 288(40), 244(50), 157(48), 140(96).

*19 & 20. 2.5-di(octylcarbamoyl) téréphtalate de di[(E)-3,7-diméthyl-2,6-octadiényle] (19), 1,3-di(octylcarbamoyl) isophtalate de di[(E)-3,7-diméthyl-2,6-octadiényle] (20)*

La synthèse a été effectuée suivant la méthode générale décrite plus haut, à partir de l'anhydride pyromellitique et du 3,7-diméthyl-2,6-octadiénol. L'anhydride mixte a été formé à l'aide du chlorure de pivaloyle. L'amine choisie était l'octylamine (sur 18,3 mmol) et le solvant $CH_2Cl_2$, TEA. On a obtenu un rendement de 77% (mélange de 19 et 20) après chromatographie (50/50, cyclohexane, acétate d'éthyle).
Données analytiques :

$^1H$ RMN($CDCl_3$, $\delta$, ppm) : 8,20(s, 1H, produit 20);7,83(s, 2H, produit 19) ;7,44(s, 1H, produit 20) ; 6,27(t, J=5,5Hz, 2H) ; 5,40(m, 2H) ; 5,09(m, 2H) ; 4,79(d, J=7,1Hz, 4H) ; 3,40(m, 4H) ; 2,16-2,03(m, 8H) ; 1,73(s, 6H) ; 1,68(s, 6H); 1,65-1,58(m, 4H) ; 1,60(s, 6H) ; 1,42-1,22(m, 20H); 0,89(t, J=6,9Hz, 6H).
$^{13}C$ RMN($CDCl_3$, $\delta$, ppm) : 167,8(s), 167,5(s), 165,6(s), 165,3(s), 143,1(s), 142,8(s), 139,0(s), 132,3(s), 131,9(d), 129,9(s), 129,2(s), 123,6(d), 117,6(d), 63,0(t), 62,9(t), 40,4(t), 39,6(t), 31,8(t), 29,4(t), 29,33(t), 29,27(t), 27,1(t), 26,3(t), 25,7(q), 22,7(t), 17,7(q), 16,6(q), 14,1(q).
SM (ESI) : 1498,1 et 1498,9(2M+$H^+$ et pic isotopique).

*21. 3-(octylcarbamoyl)propanoate de (E)-3,7-diméthyl-2,6-*octadiényle

La synthèse a été effectuée suivant la méthode générale décrite plus haut, à partir de l'anhydride succinique et du 3.7-diméthyl-2.6-octadiénol. L'anhydride mixte a été formé à l'aide du chlorure de pivaloyle. L'amine choisie était l'octylamine (sur 100 mmol) et le solvant dichlorométhane, TEA. On a obtenu un rendement de 75% après chromatographie (75/25 cyclohexane/acétate d'éthyle).
Données analytiques :

$^1H$ RMN($CDCl_3$, $\delta$, ppm) : 5,83(m, 1H) ; 5,33(m, 1H) ; 5,08(m, 1H) ; 4,61(d, J=7,1Hz, 2H) ; 3.23(~dd, J=7,1, 5,4Hz, 2H) ; 2,67(t, J=6,7Hz, 2H) ; 2,46(t, J=6,7Hz, 2H) ; 2,13-2.01 (m, 4H) ; 1,69(s, 3H) ; 1,68(s, 3H) ; 1,60(s, 3H) ; 1,52-1,43(m, 2H) ; 1,32-1.23(m, 10H) ; 0,88(t, J=6,7Hz, 3H).
$^{13}C$ RMN($CDCl_3$, $\delta$, ppm): 173,2(s), 171,4(s), 142,4(s), 131,8(s), 123,7(d), 118,2(d), 61,7(t), 39,7(t), 39,6(t), 31,8 (t), 31,2(t), 29,8(t), 29,6(t), 29,3(t), 29,2(t), 26,9(t), 26,3(t), 25,7(q), 22,7(t), 17,7(q), 16,5(q), 14,1(q).
SM (ESI) : 388,4(M+$Na^+$, 85). 230,3(100).

*22. 2-(octylcarbamoyl) benzoate de 1, 1, 5-triméthyl-6-heptényle*

La synthèse a été effectuée suivant la méthode générale décrite plus haut, à partir de l'anhydride phtalique et du 1,1,5-triméthyl-6-hepténol. Cette fois, le monophtalate a été obtenu en utilisant de la pyridine à reflux comme solvant. L'anhydride mixte a été formé à l'aide du chlorure de pivaloyle. L'amine choisie était l'octylamine (sur 10 mmol) et le solvant dichlorométhane, TEA. On a obtenu un rendement de 25% après chromatographie (80/20 cyclohexane/acétate d'éthyle).
Données analytiques :

$^1H$ RMN($CDCl_3$, $\delta$, ppm): 7,78(m, 1H) ; 7,50-7,40(m, 3H); 5,96(t élargi, J=5,3Hz, 1H) ; 5,67(ddd, J=17,6, 10,3,

7,5Hz, 1H) ; 4,94-4,89(AB de ABX, 2H) ; 3,42(m, 2H) ; 2,12(m, 1H) ; 1,84(m, 2H) : 1,60(m, 2H) ; 1,53 (s, 6H) ; 1,42-1,21(m, 14H) ; 0,98(d, J=6,7Hz, 3H) ; 0,88(t, J=6,7Hz, 3H).
$^{13}$C RMN($CDCl_3$, δ, ppm) : 169,5(s), 166,0(s), 144,6(d), 137,9(s), 131,3(d), 131,1(s), 129,8(d), 129,4(d), 127,8(d), 112,6(t), 84,6(s), 40,8(t), 40,2(t), 37,6(d), 36,8(t), 31,8(t), 29.4(t), 29,3(t), 29,2(t), 27,0(t), 25,9(q), 22,7(t), 21,6(t), 20,2(q), 14,1 (q).
SM (ESI) : 416,0(100, M+H$^+$), 278,2(95).

*23. 4-(octylcarbamoyl)butanoate de (E)-3.7-diméthyl-2,6-octadiényle*

La synthèse a été effectuée suivant la méthode générale décrite plus haut, à partir de l'anhydride glutarique et du géraniol. L'anhydride mixte a été formé à l'aide du chlorure de pivaloyle. L'amine choisie était l'octylamine (sur 10 mmol) et le solvant dichlorométhane, TEA. On a obtenu un rendement de 46% après chromatographie (8/2 cyclohexane/acétate d'éthyle).
Données analytiques :

$^1$H RMN($CDCl_3$, δ, ppm) : 5,74(t élargi, 1H) ; 5,33(m, 1H) ; 5,08(m, 1H) ; 4,59(d, J=6,7Hz, 2H) ; 3,23(~dd, J=7,1, 5,4Hz, 2H) ; 2,37(t, J=7,1Hz, 2H) ; 2,23(t, J=7,3Hz, 2H) ; 2,13-2,01(m, 4H) ; 2,00-1,92(m, 2H) ; 1,70(s, 3H) ; 1,68 (s, 3H) ; 1,60(s, 3H) ; 1,52-1,44(m, 2H) ; 1,34-1,22(m, 10H) ; 0,88(t, J=6,8Hz, 3H)
$^{13}$C RMN($CDCl_3$, δ, ppm) : 173,3(s), 172,1(s), 142,3(s), 131,8(s), 123,7(d), 118,2(d), 61,4(t), 39,5(t), 35,6(t), 33,4 (t), 31,8(t), 29,7(t), 29,3(t), 29,2(t), 27,0(t), 26,3 (t), 25,7(q), 22,7(t), 21,1 (t), 17,7(q), 16,5(q), 14,1 (q).
SM (ESI) : 380,2(M+H$^+$, 100), 244,6(80).

*24 3-(octylcarbamoyl)propanoate de (Z)-3-hexenyle*

La synthèse a été effectuée suivant la méthode générale décrite plus haut, à partir de l'anhydride succinique et du (Z)-3-hexenol. L'anhydride mixte a été formé à l'aide du chlorure de pivaloyle. L'amine choisie était l'octylamine (sur 100 mmol) et le solvant dichlorométhane, TEA. On a obtenu un rendement de 45% après chromatographie (70/30 cyclohexane/acétate d'éthyle).
Données analytiques :

$^1$H RMN($CDCl_3$, δ, ppm) : 5,83(t élargi, 1H) ; 5,50(m, 1H) ; 5,30(m, 1H) ; 4,07(d, J=7,1 Hz, 2H) ; 3,23(~dd, J=7,1, 5,3Hz, 2H) ; 2,67(t, J=6,7Hz. 2H) ; 2,46(t, J=6,9Hz, 2H); 2,37(~dd, 2H); 2,05(~dt, 2H) ; 1,52-1,44(m, 2H); 1,32-1,23 (m, 10H) ; 0.97(t, J=7,5 Hz, 3H); 0,88(t, J=6,7Hz, 3H).
$^{13}$C RMN($CDCl_3$, δ, ppm) : 173,2(s), 171,4(s), 134,7(d), 123,6(d), 64,3(t). 39.7(t), 31,8(t), 31.1(t), 29,7(t). 29,6(t), 29,3(t), 29,2(t), 26,9(t). 26,7(t). 22,7(t), 20,6(t), 14,2(q), 14,1(q).
SM (ESI) : 312.0 (100: M+H$^+$).

*25 2-(dodécylcarbamoyl)benzoate de (±)-(3-methyl-5-phenyl)pentyl*

La synthèse a été effectuée suivant la méthode générale décrite plus haut, à partir de l'anhydride phtalique et du (±)-(3-methyl-5phenyl)pentanol. L'anhydride mixte a été formé à l'aide du chlorure de pivaloyle. L'amine choisie était la dodécylamine (sur 10 mmol) et le solvant $CH_2Cl_2$, TEA. On a obtenu un rendement de 68%.
Données analytiques :

$^1$H RMN($CDCl_3$, δ, ppm) : 7,80(m, 1H) ; 7,52-7,40(m, 3H) ; 7,26-7,14(m, 5H); 5,90(t, J=5,4Hz, 1H) ; 4,39-4,26(m, 2H) ; 3,46-3,32(m, 2H) ; 2,72-2,55(m, 2H); 1,87-1,78(m, 1H) ; 1,73-1,45(m, 6H); 1,37-1,22(m, 18H) ; 1,00(d, J=6,3 Hz, 3H); 0,88(t, J=6,7Hz, 3H).
$^{13}$C RMN($CDCl_3$, δ, ppm) : 169,3(s), 166,8(s), 142,6(s), 138,5(s), 131,8(d), 130,0(d), 129, 4(d+s), 128,34(d), 128,28(d), 127,7(d), 125,6(d), 63,9(t), 40,2(t), 38,7(t), 35,2(t), 33,2(t), 31,9(t), 29,64(t), 29,60(t), 29,5(t), 29,4(t), 27,6(d+t), 22,7(t), 19,5(t), 14,1(q).
SM (CI, $NH_3$) : 516,6(30, M+$NH_4^+$), 494,7(100, M+H$^+$), 316,6(15).

*26 2,5-dioctylcarbamoyl-1,4-benzenedicarboxylate de Dioctyle*

La synthèse a été effectuée suivant la méthode générale décrite plus haut, à partir de l'anhydride pyromellitique et du 1-octanol. L'anhydride mixte a été formé à l'aide du chlorure de pivaloyle. L'amine choisie était l'octylamine (sur 10 mmol) et le solvant $CH_2Cl_2$, TEA. On a obtenu un rendement de 34% d'un seul régioisomère sous forme d'un solide blanc.

Données analytiques :

$^1H$ RMN($CDCl_3$, δ, ppm) : 7,70(s, 2H) ; 6.70(t, J=5,5 Hz, 2H) ; 4,21(t, J=6.9Hz, 4H) ; 3,39(m, 4H); 1,73-1,60(m, 8H); 1,30(m, 40H) ; 0,89(m, 12H).
$^{13}C$ RMN($CDCl_3$, δ, ppm): 167,5(s), 165,7(s), 138,8(s), 132,2(s), 129,1(d), 66,3(t), 40.4(t), 31,9(t), 29, 4(t), 29,3 (t), 29.2(t). 29.1(t), 28,5(t), 27,2(t), 25,9 (t), 22,7(t), 14,1 (q).
SM (ESI) : 764,1 (30), 701,6(100, M+H$^+$). 571,5(15).

*27 (Z)-3-dodecylcarbamoyl-2-propenoate de (E)-3, 7-dimelhyl-2, 6-octadienyle*

La synthèse a été effectuée suivant la méthode générale décrite plus haut, à partir de l'anhydride maléique et du (E)-3,7-diméthyl-2.6-octadienol. L'anhydride mixte a été formé à l'aide du chlorure de pivaloyle. L'amine choisie était la dodécylamine (sur 10 mmol) et le solvant dichlorométhane, TEA. On a obtenu un rendement de 85% après chromatographie (80/20 cyclohexane/acétate d'éthyle).
Données analytiques :

$^1H$ RMN($CDCl_3$, δ, ppm) : 8,42(t élargi, 1H) ; 6,32(d, J=13.1 Hz, 1H); 6,11 (d, J=13.1 Hz, 1H); 5,36(m, 1H) ; 5,08 (m, 1H) ; 4,70(d, J=7,1Hz, 2H); 3,31(~dd, J=7,1, 5,4Hz, 2H) ; 2,15-2,03(m, 4H); 1,73 (s, 3H); 1,68(s, 3H); 1,60(s, 3H); 1,59-1,53(m, 2H) ; 1,40-1,23(m, 18H); 0,88(t, J=6,7Hz, 3H).
$^{I3}C$ RMN($CDCl_3$, δ, ppm) : 166,4(s); 163,9(s); 143,4(s); 139,0(d); 131,9(s); 125,0(d); 123,6(d); 117,4(d); 62,4(t); 39,8(t); 39,5(t); 31,9(t); 29,7(t); 29,62(t); 29,57(t); 29,4(t); 29,3(t); 29,2(t); 27,0(t); 26,3(t); 25,7(q); 22,7(t); 17,7(q); 16,5(q); 14,1(q).
SM (ESI) : 442,3 (60; M+Na$^+$); 420,4(5, M+H$^+$); 284.3(100)

*28 3-(dodecylcarbamoyl)propanoate de (E)-3, 7-dimethyl-2, 6-octadienyle*

La synthèse a été effectuée suivant la méthode générale décrite plus haut, à partir de l'anhydride succinique et du (E)-3,'7-dimethyl-2,6-octadienol. L'anhydride mixte a été formé à l'aide du chlorure de pivaloyle. L'amine choisie était la dodecylamine (sur 10 mmol) et le solvant dichlorométhane, TEA. On a obtenu un rendement de 77% après chromatographie (80/20 cyclohexane/acétate d'éthyle).
Données analytiques :

$^1H$ RMN($CDCl_3$, δ, ppm) : 5,82(t élargi, 1H); 5,33(m, 1H); 5,08(m, 1H); 4,61(d, J=7,1Hz, 2H) ; 3,23(dd, J=7,1, 5,4Hz, 2H) ; 2,67(t, J=6,7 Hz, 2H); 2,46(t, J=6,7 Hz, 2H); 2,13-2,01(m, 4H); 1,70 (s, 3H); 1,68(s, 3H); 1,60(s, 3H); 1.52-1,44(m, 2H) ; 1,32-1,23(m, 18H); 0,88(t, J=6,7Hz, 3H).
$^{13}C$ RMN($CDCl_3$. δ, ppm).: 173,2(s); 171,4(s); 142,4(s); 131,8(s): 123,7(d); 118.2(d); 61,7(t); 39,7(t); 39,6(t); 31,9 (t); 31,2(t); 29,8(t); 29,7(t); 29,6(t); 29,4(t); 29,3(t); 26,9(t); 26,3(t); 25,7(q); 22,7(t); 17,1 (q); 16,5(q): 14,1 (q).
SM (ESI) : 444,3 (5; M+Na$^+$); 421,9(25, M+H$^+$); 286.3(100)

*29 4-(dodecylcarbamoy)butanoate de (E)-3, 7-dimethyl-2, 6-octadienyle*

La synthèse a été effectuée suivant la méthode générale décrite plus haut, à partir de l'anhydride glutarique et du (E)-3,7-dimethyl-2,6-octadienol. L'anhydride mixte a été formé à l'aide du chlorure de pivaloyle. L'amine choisie était la dodécylamine (sur 10 mmol) et le solvant dichlorométhane, TEA. On a obtenu un rendement de 49% après deux chromatographies successives (80/20 puis 75/25 cyclohexane/acétate d'éthyle).
Données analytiques :

$^1H$ RMN($CDCl_3$, δ, ppm) : 5,70(t élargi, 1H) ; 5,33(~t, J=7,1Hz, 1H); 5,08(m, 1H) ; 4,59(d, J=7,1Hz, 2H) ; 3,23(dd, J=6,7, 5,4Hz, 2H) ; 2,37(t, J=7,1 Hz, 2H); 2,22(t, J=7,5 Hz, 2H); 2,14-2,01(m, 4H); 2,00-1,92(m, 2H); 1,70 (s, 3H); 1,68(s, 3H); 1,60(s, 3H); 1,52-1,44(m, 2H) ; 1,32-1,23(m, 18H); 0,88(t, J=7,1 Hz, 3H).
$^{13}C$ RMN($CDCl_3$, δ, ppm) : 173,3(s); 172,1(s); 142,3(s); 131,9(s); 123,7(d); 118,2(d); 61,4(t); 39,6(t); 35,6(t); 33,4 (t); 31,9(t); 29,7(t); 29,61(t); 29,57(t); 29,4(t); 29,3(t); 27,0(t); 26,3(t); 25,7(q); 22,7(t); 21,1(t); 17,7(q); 16,5(q); 14,1 (q).
SM (ESI) : 458,3 (5; M+Na$^+$); 436,0(5, M+H$^+$); 300,3(75)

*30 2-(dodecylcarbainoyl)benzoate de 3-phenypropyle*

La synthèse a été effectuée suivant la méthode générale décrite plus haut, à partir de l'anhydride phtalique

et du 3-phenyl-1-propanol. L'anhydride mixte a été formé à l'aide du chlorure de pivaloyle. L'amine choisie était la dodécylamine (sur 10 mmol) et le solvant dichlorométhane, TEA. On a obtenu un rendement de 67% après chromatographie (60/40 cyclohexane/acétate d'éthyle).
Données analytiques :

$^{1}H$ RMN($CDCl_3$, δ, ppm): 7,82(m, 1H); 7,52-7,41 (m, 3H); 7.29-7.25(m. 2H); 7.21-7.16(m. 3H); 5,99(t, J=5,2 Hz, 1H); 4,30(t. J=6,5 Hz, 2H); 3,40(dd, J=7,1 ,5,2 Hz, 2H); 2,74(m, 2H); 2.09-2,01(m, 2H); 1,62-1,53(m. 2H); 1,36-1.22 (m, 18H); 0,88(t, J=6,7 Hz, 3H).
$^{13}C$ RMN($CDCl_3$, δ, ppm) : 169,3(s); 166,8(s); 141,2(s); 138,4(s); 131,8(d); 130,0(d); 129,5(d); 129,4(s); i 28,4(d); 127,7(d); 126,0(d); 64,9(t); 40,2(t); 32,2(t); 31,9(t); 30,1 (t); 29,7(t); 29,6(t); 29,5(t); 29,4(t); 27,0(t); 22,7(t); 14,1(q).
SM (ESI) : 452,2(100, $M+H^+$).

*31 2-(dodecylcarbamoyl)benzoate de (±)-3,7-dimethyloctyle*

La synthèse a été effectuée suivant la méthode générale décrite plus haut, à partir de l'anhydride phtalique et du (±)-3,7-dimethyloctanol. L'anhydride mixte a été formé à l'aide du chlorure de pivaloyle. L'amine choisie était la dodecylamine (sur 10 mmol) et le solvant dichlorométhane, TEA. On a obtenu un rendement de 77% après chromatographie (80/20 cyclohexane/acétate d'éthyle).
Données analytiques :

$^{1}H$ RMN($CDCl_3$, δ, ppm) : 7,84(m, 1H); 7,52-7,42(m, 3H); 6,01(t, J=5,5 Hz, 1H); 4,31(m, 2H); 3,41 (dd, J=7,1 1 , 5,5 Hz, 2H); 1,76(m, 1H); 1,64-1,45(m, 5H); ; 1,37-1,25(m, 22H); 1,18-1,11(m, 2H); 0,93(d, J=6,3 Hz, 3H); 0,88(t, J=6,8 Hz, 3H); 0,87(d, J=6,7 Hz, 6H).
$^{13}C$ RMN($CDCl_3$, δ, ppm) : 169,3(s); 166,9(s); 138,5(s); 131,7(d); 129,9(d); 129,5(s); 129,4(d); 127,7(d); 64,2(t); 40,2(t); 39,2(t); 37,2(t); 35,4(t); 31,9(t); 29,9(d); 29,6(t); 29,5(t); 29,4(t); 28,0(d); 27,1(t); 24,6(t); 22,7(q); 22,6(q); 19,6(q); 14,1(q).
SM (ESI) : 474,1 (100, $M+H^+$); 316,2(30).

*32 2-(isopropylcarbamoyl)benzoate de 2-phényléthyle*

La synthèse a été effectuée à partir du dichlorure de phtaloyle et du phényléthanol. Du phényléthanol (5 g, 41 mmol) en solution dans 40 ml de dichlorométhane a été ajouté à 0°C à un mélange de dichlorure de phtaloyle (1 equiv.) et de TEA (1 equiv.) dans 40 ml de dichlorométhane. Après 2 h de réaction, on a ajouté une solution d'isopropylamine (1 equiv.) et de la TEA (1 equiv.) dans 20 ml de dichlorométhane, puis on a laissé agiter pendant 2 h. Après les traitements habituels, on a obtenu un rendement de 51% après recristallisation dans l'éther diéthylique.
Données analytiques :

$^{1}H$ RMN($CDCl_3$, δ, ppm) : 7,75(m, 1H) ; 7,48-7,36(m, 3H) ; 7,32-7,18(m, 5H); 5,87(d large, J=7.9Hz, 1H) ; 4,47(t, J=7,3Hz, 2H); 4,22(heptuplet de doublets, J=7,9, 6,7 Hz. 1H); 3,02(t, J=7,3 Hz, 2H) ; 1,22(d, J=6,7Hz, 6H).
$^{13}C$ RMN($CDCl_3$, δ, ppm) : 168,4(s), 166,6(s), 138,6(s), 137,6(s), 131,7(d), 129,9(d), 129,3(d), 128,9(d), 128,5(d), 127,6(d), 126,6(d), 65,9(t), 41,9(d), 35,0(t), 22,6(q).
SM (CI, $NH_3$) : 329(100, $M+NH_4^+$), 312(95, $M+H^+$), 244(12), 207(20), 140(55), 117(35).

*33 2-(isopropylcarbamoyl)benzoate de (3R)-3, 7-diméthyl-6-octényle*

La synthèse a été effectuée à partir du dichlorure de phtaloyle et du (3R)-3,7-diméthyl-6-octénol sur 32 mmol selon le procédé décrit plus haut. On a obtenu un rendement de 51 % après chromatographie (75/25 cyclohexane/ acétate d'éthyle).
Données analytiques :

$^{1}H$ RMN($CDCl_3$, δ, ppm): 7,82(m, 1H) ; 7,51-7,40(m, 3H); 5,84(d large, J=7.8Hz, 1H) ; 5,09(m, 1H); 4,38-4,18(m, 3H); 2,08-1,90(m, 3H); 1,83-1,73(m, 1H); 1,67(s, 3H); 1,65-1,44(m, 1H); 1,59(s, 3H); 1,44-1,33(m, 1H); 1,25(d, J=6,7 Hz, 6H); 1,25-1,17(m, 1H); 0,95(d, J=6,4 Hz, 3H).
$^{13}C$ RMN($CDCl_3$, δ, ppm) : 168,5(s), 166,8(s), 138,5(s), 137,6(s), 131,7(d), 129,9(d), 129,6(d); 129,4(d), 127,7(d), 124,6(d), 64,1(t), 42,0(d), 37,0(t), 35,3(t); 29,5(d); 25,7(q); 25,4(t); 22,6(q); 19,5(q); 17,7(q).
SM (EI) : 346(5, M+1); 208(100); 190(95); 148(75); 130(25); 123(20); 95(25); 81 (30); 69(40); 60(25); 41 (40).

*34 2-(2-dodecylcarbamoyl)benzoate de (E)-3, 7-diméthyl-2, 6-octadiényle*

La synthèse a été effectuée suivant la méthode générale décrite plus haut, à partir de l'anhydride phtalique et du 3,7-diméthyl-2,6-octadiénol. L'anhydride mixte a été formé à l'aide de chloroformiate d'éthyle. L'amine choisie était la 2-dodécylamine (sur 20 mmol) et le solvant dichlorométhane, TEA. On a obtenu un rendement de 60% après chromatographie (80/20 cyclohexane/acétate d'éthyle).
Données analytiques :

$^1$H RMN($CDCl_3$, $\delta$, ppm) : 7,85(m, 1H) ; 7,52-7,41 (m, 3H); 5,71(d, J=8,3 Hz, 1H) ; 5,43(dt, J=7,1 1,1 Hz, 1H) ; 5,09(t élargi, J=6,7 Hz, 1H) ; 4,81(d, J=7,1 Hz, 2H) ; 4,14(~hept, J=6,7 Hz; 1H) ; 2,15-2,02(m, 4H) ; 1,74(s, 3H) ; 1,68(s, 3H); 1,60(s, 3H); 1,57-1,45 (m, 2H); 1,43-1,23(m, 16H); 1,23(d, J=6,7 Hz, 3H); 0,88(d, J=6,7Hz, 3H).
$^{13}$C RMN($CDCl_3$, $\delta$, ppm) : 168,5(s); 166,8(s); 142,3(s); 138,5(s); 131,8(s); 131,7(d); 130,0(d); 129,6(s); 129,4(d); 127,7(d); 123,8(d); 118,2(d); 62,5(t); 45,9(d); 39,6(t); 36.9(t); 26,3(t); 26,1(t); 25,7(q), 22,7(t); 20,7(q); 17,7(q), 16,6 (q); 14,1(q).
SM (CI, $NH_3$) : 487(35, $M+NH_4^+$); 470(100, $M+H^+$), 351(30), 334(60).

*35 2-(2-dodécylcarbamoyl)benzoate de 2-phényléthyle*

La synthèse a été effectuée suivant la méthode générale décrite plus haut, à partir de l'anhydride phtalique et du 2-phényléthanol. L'anhydride mixte a été formé à l'aide de chloroformiate d'éthyle. L'amine choisie était la 2-dodécylamine (sur 20 mmol) et le solvant dichlorométhane, TEA. On a obtenu un rendement de 52% après chromatographie (80/20 cyclohexane/acétate d'éthyle).
Données analytiques :

$^1$H RMN($CDCl_3$, $\delta$, ppm) : 7,77(m, 1H) ; 7,50-7,38(m, 3H) ; 7,32-7,19(m, 5H); 5,73(d, J=8,3 Hz, 1H); 4,48(t, J=7,3 Hz, 2H); 4,14(~hept, J=6,5 Hz, 1H); 3,04(t, J=7,3 Hz, 2H); 1,59-1,45(m, 2H); 1,43-1,34(m, 2H); 1,31-1,22(m, 16H); 1,22(d, J=6,5 Hz, 3H); 0,88(t, J=6,7 Hz, 3H).
$^{13}$C RMN($CDCl_3$, $\delta$, ppm) : 168,5(s); 166,6(s); 138,7(s); 137,6(s); 131,8(d); 129,9(d); 129,4(d); 129,0(d); 128,5(d); 127,6(d); 126,6(d); 65,9(t); 45,9(d); 36,8(t); 35,0(t); 31,9(t); 29,6(t); 29,3(t); 26,1(t); 22,7(q); 20,7(q); 14,1(q).
SM (EI) : 437(5, $M^+$); 332(10); 315(5);; 297(5); 184(15); 174(30); 148(10); 130(10); 105(100); 91(10); 79(7); 44(15).

*36 2-(2-dodécylcarbamoyl)benzoate de (3R)-3, 7-diméthyl-6-octényle*

La synthèse a été effectuée suivant la méthode générale décrite plus haut, à partir de l'anhydride phtalique et du (3R)-3,7-diméthyl-6-octénol. L'anhydride mixte a été formé à l'aide de chloroformiate d'éthyle. L'amine choisie était la 2-dodécylamine (sur 20 mmol) et le solvant dichlorométhane, TEA. On a obtenu un rendement de 80% après chromatographie (80/20 cyclohexane/acétate d'éthyle).
Données analytiques :

$^1$H RMN($CDCl_3$, $\delta$, ppm): 7,82(m, 1H) ; 7,52-7,41(m, 3H); 5,74(d, J=8,7 Hz, 1H) ; 5,09(m, 1H) ; 4,38-4,26(m, 2H); 4,20-4,08(m, 1H) ; 2,08-1,91(m, 2H); 1,84-1,74(m, 1H) ; 1,67(s, 3H) ; 1,60(s, 3H) ; 1,63-1,45 (m, 2H); 1,42-1,15 (m, 20H); 1,23(d, J=6,7 Hz, 3H); 0,95(d, J=6,3 Hz, 3H); 0,88(d, J=6,7Hz, 3H).
$^{13}$C RMN($CDCl_3$, $\delta$, ppm): 168,5(s); 166,9(s); 138,6(s); 131,7(d); 131,3(s); 129,9(d); 129,7(s); 129,4(d); 127,7(d); 124,6(d); 64,1 (t); 45,9(d); 37,0(t); 36,9(t); 35,4(t); 31,9(t); 29,64(t); 29,56(t); 29,4(t); 26,1(t); 25,7(q), 25,4(t); 22,7 (t); 20,7(q); 19,5(q); 17,7(q);14,1(q).
SM (EI) : 471(35, $M^+$); 334(100), 316(40), 186(10); 174(35); 148(70); 139(20); 95(20); 83(40); 69(85); 55(40); 41 (45).

<u>Exemple 2</u>

<u>Essais de libération des alcools odorants en milieu basique</u>

Plusieurs essais ont été effectués à pH 7,6 et 10,2 sur des composés de l'invention pour contrôler l'hydrolyse de la fonction ester, selon la méthode générale suivante.

**Méthode générale**

Généralités : A t = 0, on a ajouté rapidement 1 ml d'une solution (1,6 mg/ml) de composé de l'invention dans

l'acétonitrile à une solution tampon (eau/acétonitrile 4:1) à pH égal à 7,6. respectivement 10,2. La solution tampon a été préparée en diluant deux pastilles tampon de phosphate (pH=7,6) ou borate (pH=10,2) (Fluka) dans un mélange de 160 ml d'eau et 40 ml d'acétonitrile. L'hydrolyse a été suivie par HPLC (High Pressure Liquid Chromatography) à 20°C jusqu'à ce que la libération soit achevée. On a ainsi vérifié à l'aide de la méthode citée (HPLC) l'hydrolyse avec libération d'un alcool odorant des composés 1 à 36dans les conditions de pH susmentionnées.

Exemple 3

Essais sur textiles avec l'appareil *Linitest*

Protocole pour une lavette: Une lavette pesée (28 cm x 28 cm, environ 36 g) est placée dans un container en acier Inox de 600 ml avec 1,8 g de détergent standard (Henkel, ECE Colour Fastness Test Detergent 77) et 400 ml d'eau. Le container est agité dans un bain Marie rotatif *Linitest* à 42°C pendant 20 minutes. La lavette a ensuite été rinçée 2 fois avec 600 ml d'eau. Le rinçage adoussissant a été effectué avec 600 ml d'eau contenant 1,8 g de base adoussissante concentrée trois fois (référence 91/28 composition donnée ci-dessous exemple 3). Cette base contenait 0.8 % en poids de précurseur de parfum ou bien l'alcool libre correspondant en quantité équimolaire. La lavette a ensuite été essorée à la main à poids constant (70-75 g). Des lavettes traitées avec chaque précurseur et celles traitées avec l'alcool parfumant correspondant ont été comparées olfactivement par un panel de 4 personnes après différents temps de séchage à l'air libre. Ainsi on a pu démontrer que la plupart des composés décrits dans les points 1-36 est au moins aussi efficace que les alcools libres correspondants. En général, les composés de l'invention restent odorants plus longtemps et avec une intensité olfactive en moyenne plus grande.

Exemple 4

Essai sur textiles

Plusieurs tests ont été exécutés sur des textiles, dans des conditions variées, ces textiles ayant été traités selon la méthode générale suivante.

**Méthode générale de traitement des textiles**

Environ 1 kg de serviettes éponge standard de 28x28 cm a été lavé à 40° dans une machine à laver (Miele, modèle Deluxe electronic W724), sans prélavage, en utilisant 50 g d'une base standard de détergent (Henkel, ECE Colour Fastness Test Detergent 77) et 50 g d'adoucissant non parfumé courant contenant des Ester Quats (HEQ).

La base adoucissante textile utilisée avait la composition suivante :

| Ingrédients | % en poids |
|---|---|
| Mélange HEQ-Esterquat/acide gras $C_{16}$-$C_{18}$ (6 :1) | 14 |
| Ethoxylate de suif de noix de coco 20EO | 0,75 |
| Alcool de suif | 0,75 |
| Eau | 84,5 |
| Total | 100,00 |

Dans deux essais séparés, des serviettes éponge ont été traitées selon cette méthode générale, en utilisant en tant qu'additif de l'adoucissant textile, respectivement du 2-(octylcarbamoyl)benzoate de géranyle, le composé 5 (0,76% en poids) dans l'essai A et du géraniol (0,28% en poids) dans l'essai B. Les 2 groupes de serviettes ont été soumis à un panel d'experts pour évaluation à l'aveugle à la sortie de la machine à laver et 24 h après. Sur linge mouillé les serviettes traitées dans l'essai A semblaient beaucoup plus odorantes que celles traitées dans l'essai B. La même chose a été constatée, 24 h après le lavage. L' odeur a en outre perduré durant 3 jours après le lavage.

Exemple 5

Essai sur textiles

Deux groupes de serviettes éponge standard ont été traités séparément et de façon identique comme il est décrit dans l'Exemple 3, le seul élément changeant étant l'additif incorporé dans la base adoucissante, à savoir du

2-(dodécylcarbamoyl)benzoate de géranyle dans l'essai A, et du géraniol dans l'essai B.
De même, une évaluation 24 h après le lavage de chacun des groupes de serviettes a montré que l'odeur de l'alcool était à la fois plus intense dans le cas de l'essai A et perceptible pendant bien plus longtemps.

Exemple 6

Essai sur textiles

Deux groupes de serviettes éponge standard ont été traités séparément et de façon identique comme il est décrit dans l'Exemple 3, le seul élément changeant étant l'additif incorporé dans la base adoucissante, à savoir de 3-(octylcarbamoyl)propanoate de géranyle dans l'essai A, et du géraniol dans l'essai B. 24 h après le lavage et durant encore 4 jours, les serviettes traitées dans l'essai A dégageaient encore l'odeur de l'alcool contrairement à celles traitées dans l'essai B.

Exemple 7

Essai sur textiles

Deux groupes de serviettes éponge standard ont été traités séparément comme il est décrit dans l'Exemple 3, le seul élément changeant étant un mélange de 3 additifs incorporés dans la base adoucissante, à savoir un mélange de 2-dodecylcarbamoyl benzoate de géranyle, de 2-dodecylcarbamoyl benzoate de citronellyl et 2-dodecylcarbamoyl benzoate de phényléthyle dans l'essai A, et une quantité correspondante d'un mélange de géraniol, citronellol et phénylethanol dans les mêmes proportions dans l'essai B. L'intensité olfactive des serviettes de l'essai A était bien plus forte que celles des serviettes de l'essai B. De plus il a été trouvé, olfactivement et analytiquement, par GC-SPME (gas chromatography - solid phase micro extraction) que l'équilibre des trois alcools odorants était mieux respecté dans le cas de l'essai A que dans celui de l'essai B. Cet exemple démontre que le transfert des précurseurs de parfums de notre invention depuis la base adoucissante vers le linge se fait de façon plus optimale que le transfert des alcools odorants libres correspondants. Cet exemple montre également l'intérêt d'utiliser les précurseurs de l'invention afin de relarguer directement non plus un composé seul mais un mélange de composés formant un *accord* de parfumerie.

**Revendications**

1. Composition parfumante ou article parfumé contenant des ingrédients parfumants, solvants et, ou, adjuvants couramment utilisés pour la préparation de parfums et articles parfumés et, en tant qu'ingrédient actif, un composé de formule

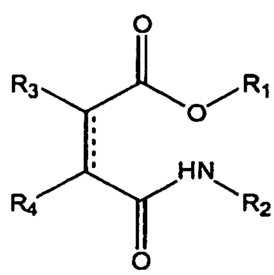

ou

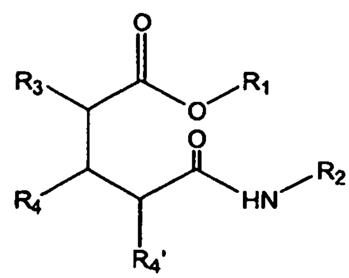

(I)

dans laquelle la ligne pointillée indique l'emplacement d'une liaison simple ou double ; $R_1$ représente un radical dérivé d'un alcool couramment utilisé à titre d'ingrédient parfumant pour la préparation de parfums ou d'articles parfumés, de formule $R_1OH$ ; $R_2$ représente un atome d'hydrogène, un radical d'hydrocarbure linéaire ou ramifié, saturé ou insaturé, de $C_1$ à $C_{30}$, ou un cycle aliphatique ou aromatique ayant 5 ou 6 atomes de carbone, ce radical $R_2$ pouvant comporter des hétéroatomes d'oxygène, de soufre ou d'azote ; les symboles $R_3$, $R_4$ et $R_4$' pris indépendamment, représentent un atome d'hydrogène, un radical d'hydrocarbure linéaire ou ramifié, saturé ou insaturé, le cas échéant substitué, de $C_1$ à $C_{20}$ pouvant comporter un ou plusieurs hétéroatomes ou, lorsque pris ensemble avec les atomes de carbone auxquels ils sont liés, peuvent former des monocycles, bicycles ou tricycles

aromatiques ou aliphatiques, les radicaux $R_3$, $R_4$ et $R_4$' pouvant comprendre des groupes fonctionnels de type ester et carbamoyle.

**2.** Composition parfumante ou article parfumé selon la revendication 1, charctérisée en se que le radical dérivé d'un alcool de formule $R_1OH$ est choisi parmi l'alcool anisique, l'alcool cinnamique, l'alcool fenchylique, le 9-décén-1-ol, le phénéthylol, le citronellol (3,7-diméthyl-6-octén-1-ol), le 3-méthyl-5-phényl-1-pentanol, le 7-p-menthan-1-ol , le dihydromyrcénol (2,6-diméthyl-oct-7-én-2-ol), le alpha-ionol, le tétrahydro-ionol, le géraniol, le nérol, le (Z)-3-hexén-1-ol, le 1-hexanol, le 3,3,5-triméthyl-hexanol, le 3,4,5,6,6-pentaméthyl-heptan-2-ol, le 5-éthyl-2-nonanol, le cis-6-nonénol, le 6,8-diméthyl-2-nonanol, le 2,6-nonadièn-1-ol, le bornéol, le 1-octèn-3-ol, le 4-cyclohexyl-2-méthyl-2-butanol, le 6-éthyl-3-méthyl-5-octèn-1-ol, le 3,7-diméthyl-oct-3,6-diénol, le 7-méthoxy-3,7-diméthyl-octan-2-ol, le 2-méthyl-1-phényl-2-propanol, le 1-phényléthanol, le 2-phényléthanol, le 2-phénylpropanol, le 3-phénylpropanol, le 2-méthyl-5-phénylpentanol, le 2-méthyl-4-phénylpentanol, le 3-méthyl-5-phénylpentanol, le cyclométhylcitronellol, le décanol, le dihydroeugénol, le 8-p-menthanol, le 3,7-diméthyl-1-octanol, le 2,6-diméthyl-2-heptanol, le dodécanol, l'octanol, l'undécanol, le 4-méthyl-3-décèn-1-ol, l'eugénol, le tétrahydro-2-isobutyl-4-méthyl-4(2H)-pyranol, le 2-phénoxy-éthanol, l'isoeugénol, le linalol, le 2-méthoxy-4-propyl-1-cyclohexanol , la vanilline, l'éthyl-vanilline, le farnésol, le cédrénol, le menthol, le p-menth-8-èn-3-ol, le 3,3,5-triméthyl-cyclohexanol, le 2,4,6-triméthyl-3-cyclohexényl-méthanol, le 4-(1-méthyléthyl)cyclohexyl-méthanol, le terpinéol, le tétrahydromuguol, le 3,7-diméthyl-3-octanol, le (E)-3,3-diméthyl-5-(2',2',3'-triméthyl-3'-cyclopentèn-1-yl)-4-pentèn-2-ol , le 2,2,6-triméthyl-alpha-propyl-cyclohexane propanol, le 5-(2,2,3-triméthyl-3-cyclopentyl)-3-méthylpentan-2-ol, le 3-méthyl-5-(2,2,3-triméthylcyclo-pentyl-3-ényl)pent-4-èn-2-ol, le 2-éthyl-4(2,2,3-triméthylcyclopentyl-3-ényl)but-2-èn-1-ol, le 4-(5,5,6-triméthylbicyclo[2.2.1]hept-2-yl)-cyclohexanol, le 2-(2-méthylpropyl)-4-hydroxy-4-méthyl-tétrahydropyrane, le 2-cyclohexyl propanol, le 2-(1,1-diméthyléthyl)-4-méthyl-cyclohexanol, le 1-(2-tert-butylcyclohexyloxy)-2-butanol, le 1-(4-isopropyl-cyclohexyl)-éthanol et le 1-(2,2,3,6-tétraméthyl-cyclohex-1-yl)-3-hexanol.

**3.** Composition parfumante ou article parfumé contenant en tant qu'ingrédient actif un composé de formule

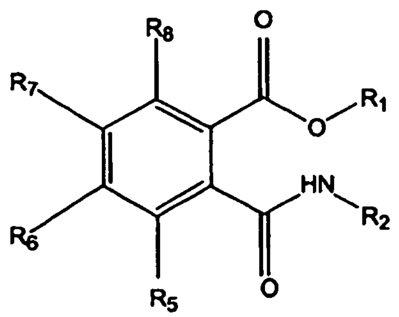

(Ia)

dans laquelle les symboles $R_1$ et $R_2$ sont définis comme à la revendication 1 ; $R_5$, $R_6$, $R_7$ et $R_8$ pris indépendamment, représentent chacun un atome d'hydrogène ou un radical allcyle linéaire ou ramifié de $C_1$ à $C_{20}$, ou, pris deux à deux, peuvent former un ou plusieurs cycles avec les atomes de carbone auxquels il sont liés.

**4.** Composition ou article selon la revendication 3, **caractérisé en ce que** l'ingrédient actif est un composé choisi dans le groupe constitué par le 2-(octylcarbamoyl)benzoate de 3,7-diméthyl-6-octényle, le 2-(dodécylcarbamoyle) benzoate de 3,7-diméthyl-2,6-octadiényle et le 2-(dodécylcarbamoyl)benzoate de 3,7-diméthyl-6-octényle.

**5.** Composition parfumante ou article parfumé contenant en tant qu'ingrédient actif un composé de formule

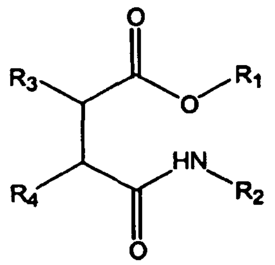

ou

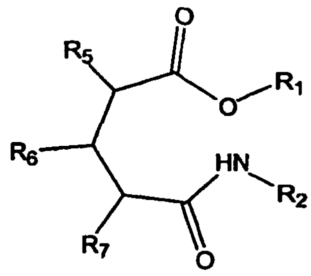

(Ib) (Ic)

dans lesquelles les symboles $R_1$ et $R_2$ sont définis comme à la revendication 1 ; $R_3$, $R_4$, $R_5$, $R_6$, $R_7$ représentent chacun un atome d'hydrogène ou un radical alkyle linéaire ou ramifié de $C_1$ à $C_{20}$ pouvant contenir un ou plusieurs hétéroatomes.

6. Composition ou article selon la revendication 5, **caractérisé en ce que** l'ingrédient actif est un composé choisi dans le groupe constitué par le 3-(octylcarbamoyl)propanoate de 3-phényl-2-propényle, le 4-(octylcarbamoyl)butanoate de 3,7-diméthyl-2,6-octadiényle et le 3-(octylcarbamoyl)propanoate de 3,7-diméthyl-2,6-octadiényle.

7. Composition parfumante ou article parfumé contenant en tant qu'ingrédient actif un composé de formule

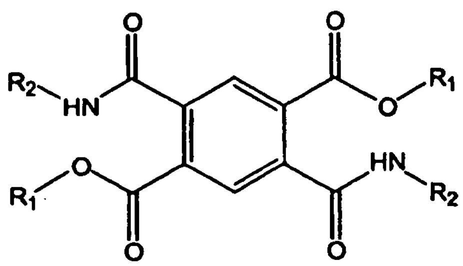

ou

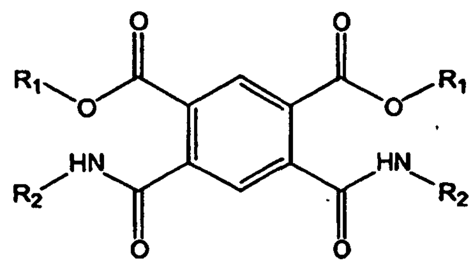

(Id)

dans lesquelles les symboles $R_1$ et $R_2$ sont définis comme à la revendication 1.

8. Composition ou article selon la revendication 7, **caractérisé en ce que** l'ingrédient actif est un composé choisi parmi le 2,5-di(octylcarbamoyl)téréphtalate de di(3,7-diméthyl-2,6-octadiényl), le 1,3-di(octylcarbamoyl)isophtalate de di(3,7-diméthyl-2,6-octadiényl), ou un mélange de ces deux composés.

9. Article parfumé selon l'une des revendications 1 à 8, sous la forme d'une lotion après-rasage, d'une préparation cosmétique, d'un savon, d'un shampooing ou après-shampooing ou autre produit de soin capillaire, d'un gel de bain ou de douche, d'un déodorant corporel ou d'un désodorisant ambiant, d'un détergent ou adoucissant textile, ou d'un produit d'entretien.

10. Utilisation d'un composé tel que défini dans l'une des revendications 1 à 8, à titre de précurseur susceptible de libérer un alcool parfumant.

11. Utilisation selon la revendication 10, **caractérisée en ce que** le résidu dudit composé après libération de l'alcool parfumant est inodore.

12. Procédé pour le parfumage de textiles soumis à un traitement de lavage en présence d'un détergent, suivi facultativement d'un traitement avec un adoucissant textile, le procédé étant **caractérisé en ce que** les textiles sont traités en présence d'un composé tel que défini à la revendication 1.

**13.** Procédé selon la revendication 12, **caractérisé en ce que** le composé selon la revendication 1 est compris dans le détergent et/ou adoucissant.

**14.** Procédé pour intensifier ou prolonger l'effet de diffusion de l'odeur caractéristique d'un alcool odorant développée par des textiles, **caractérisé en ce qu'**on soumet ces textiles à un cycle de lavage en présence d'un détergent et, facultativement à un traitement subséquent avec un adoucissant textile, ledit détergent et/ou adoucissant contenant un composé tel que défini à la revendication 1.

**15.** Composé de formule (Ia) telle que définie à la revendication 3, choisi dans le groupe constitué par le 2-(octylcarbamoyl)benzoate de 3,7-diméthyl-6-octényle, le 2-(dodécylcarbamoyle)benzoate de 3,7-diméthyl-2,6-octadiényle et le 2-(dodécylcarbamoyl)benzoate de 3,7-diméthyl-6-octényle.

**16.** Composé de formule (Ib) ou (Ic) telles que définies à la revendication 5, choisi dans le groupe constitué par le 3-(octylcarbamoyl)propanoate de 3-phényl-2-propényle, le 4-(octylcarbamoyl)butanoate de 3,7-diméthyl-2,6-octadiényle et le 3-(octylcarbamoyl)propanoate de 3,7-diméthyl-2,6-octadiényle.

**17.** Composé de formule

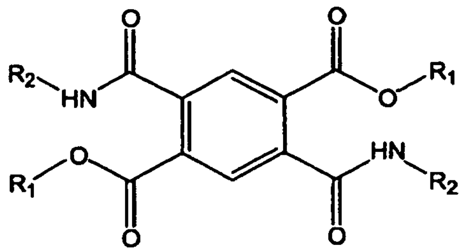

ou

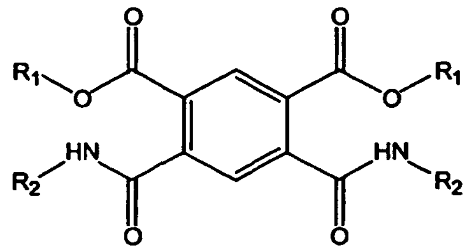

(Id)

dans lesquelles les symboles $R_1$ et $R_2$ sont définis comme à la revendication 1.

**18.** Composé selon la revendication 17, choisi parmi le 2,5-di(octylcarbamoyl)téréphtalate de di(3,7-diméthyl-2,6-octadiényl), le 1,3-di(octylcarbamoyl)isophtalate de di(3,7-diméthyl-2,6-octadiényl), ou un mélange de ces deux composés.

## Claims

**1.** A perfuming composition or perfumed article comprising perfuming ingredients, solvents, and/or adjuvants of current use in the preparation of perfumes and perfumed products and, as an active ingredient, a compound of formula

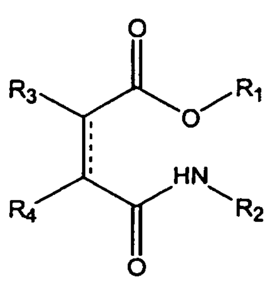

or

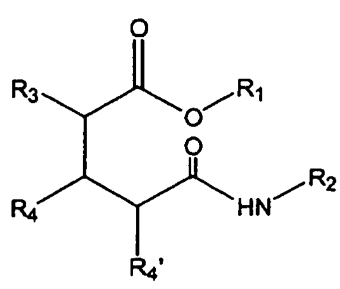

(I)

in which the dotted line indicates the location of a single or double bond; $R_1$ represents a radical derived from an alcohol of current use as perfuming ingredient in the preparation of perfumes or perfumed articles, of the formula $R_1OH$; $R_2$ represents a hydrogen atom, a linear or branched, saturated or unsaturated $C_1$-$C_{30}$ hydrocarbon radical, or an aliphatic or aromatic cyclic compound having 5 or 6 carbon atoms, this radical $R_2$ possibly comprising het-

eroatoms of oxygen, sulphur or nitrogen; the symbols $R_3$, $R_4$ and $R_4'$, considered independently, represent a hydrogen atom, a linear or branched, saturated or unsaturated, optionally substituted, $C_1$ to $C_{20}$ hydrocarbon radical possibly comprising one or more heteroatoms, or, when considered together with the carbon atoms to which they are bonded, can form aromatic or aliphatic monocyclic, bicyclic or tricyclic compounds, the radicals $R_3$, $R_4$ and $R_4'$ possibly comprising functional groups of the ester and carbamoyl type.

**2.** The perfuming composition or perfumed article according to claim 1, **characterised in that** the radical derived from an alcohol of the formula $R_1OH$ is selected from anisyc alcohol, cinnamic alcohol, fenchyl alcohol, 9-decen-1-ol, phenethylol, citronellol (3,7-dimethyl-6-octen-l-ol), 3-methyl-5-phenyl-l-pentanol, 7-p-menthan-1-ol, dihydromyrcenol (2,6-dimethyl-oct-7-en-2-ol), alpha-ionol, tetrahydro-ionol, geraniol, nerol, (Z)-3-hexen-1-ol, 1-hexanol, 2-hexanol, 3,3,5-trimethyl-hexanol, 3,4,5,6,6-pentamethyl-heptan-2-ol, 5-ethyl-2-nonanol, *cis*-6-nonenol, 6,8-dimethyl-2-nonanol, 2,6-nonadien-1-ol, bomeol, 1-octen-3-ol, 4-cyclohexyl-2-methyl-2-butanol, 6-ethyl-3-methyl-5-octen-1-ol, 3,7-dimethyl-oct-3,6-dienol, 7-methoxy-3,7-dimethyl-octan-2-ol, 2-methyl-1-phenyl-2-propanol, 1-phenylethanol, 2-phenylethanol, 2-phenylpropanol, 3-phenylpropanol, 2-methyl-5-phenylpentanol, 2-methyl-4-phenylpentanol, 3-methyl-5-phenylpentanol, cyclomethylcitronellol, decanol, dihydroeugenol, 8-p-menthanol, 3,7-dimethyl-1-octanol, 2,6-dimethyl-2-heptanol, dodecanol, octanol, undecanol, 4-methyl-3-decen-1-ol, eugenol, tetrahydro-2-isobutyl-4-methyl-4(2H)-pyranol, 2-phenoxy-ethanol, isoeugenol, linalol, 2-methoxy-4-propyl-1-cyclohexanol, vanillin, ethyl-vanillin, farnesol, cedrenol, menthol, p-menth-8-en-3-ol, 3,3,5-trimethyl-cyclohexanol, 2,4,6-trimethyl-3-cyclohexenyl-methanol, 4-(1-methylethyl)cyclohexyl-methanol, terpineol, tetrahydromuguol, 3,7-dimethyl-3-octanol, (E)-3,3-dimethyl-5-(2',2',3'-trimethyl-3'-cyclopenten-1-yl)-4-penten-2-ol, 2,2,6-trimethyl-alpha-propyl-cyclohexane propanol, 5-(2,2,3-trimethyl-3-cyclopentyl)-3-methylpentan-2-ol, 3-methyl-5-(2,2,3-trimethylcyclopentyl-3-enyl)pent-4-en-2-ol, 2-ethyl-4(2,2,3-trimethylcyclo-pentyl-3-enyl)but-2-en-1-ol, 4-(5,5,6-trimethylbicyclo[2.2.1]hept-2-yl)-cyclohexanol, 2-(2-methyl-propyl)-4-hydroxy-4-methyltetrahydropyran, 2-cyclohexyl-propanol, 2-(1,1-dimethylethyl)-4-methyl-cyclohexanol, 1-(2-tert-butyl-cyclo-hexyloxy)-2-butanol, 1-(4-isopropyl-cyclohexyl)-ethanol and 1-(2,2, 3, 6-tetramethyl-cyclohex-1-yl)-3-hexanol.

**3.** A perfuming composition or perfumed article comprising, as an active ingredient, a compound of formula

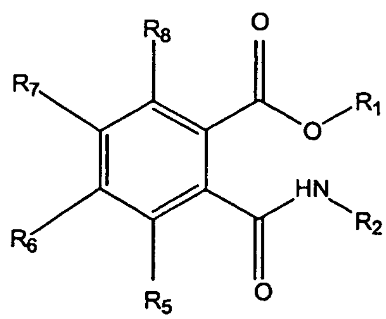

(Ia)

in which the symbols $R_1$ and $R_2$ are as defined for claim 1; $R_5$, $R_6$, $R_7$ and $R_8$, considered independently, each represent a hydrogen atom or a linear or branched $C_1$ to $C_{20}$ alkyl radical, or, when taken in pairs, can form one or more cyclic compounds with the carbon atoms to which they are bonded.

**4.** A composition or an article according to claim 3, **characterised in that**, the active ingredient is selected from the group consisting of 2-(octylcarbamoyl)benzoate of 3,7-dimethyl-6-octenyl, 2-(dodecyl-carbamoyl)benzoate of 3,7-dimethyl-2,6-octadienyl and 2-(dodecyl-carbamoyl)benzoate of 3,7-dimethyl-6-octenyl).

**5.** A perfuming composition or perfumed article comprising, as an active ingredient, a compound of formula

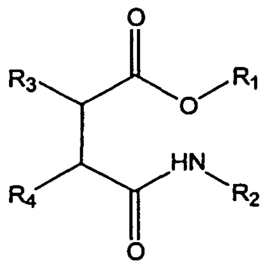

or

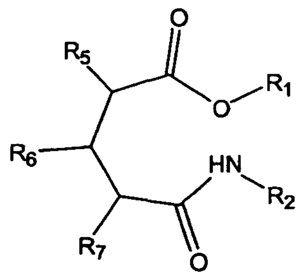

(Ib) (Ic)

in which the symbols $R_1$ and $R_2$ are as defined for claim 1; $R_3$, $R_4$, $R_5$, $R_6$ and $R_7$ each represent a hydrogen atom or a linear or branched $C_1$ to $C_{20}$ alkyl radical possibly containing one or more heteroatoms.

**6.** A composition or an article according to claim 5, **characterised in that** the active ingredient is a compound selected from the group consisting of 3-(octylcarbamoyl)propanoate of 3-phenyl-2-propenyl, 4-(octylcarbamoyl)butanoate of 3,7-dimethyl-2,6-octadienyl and 3-(octylcarbamoyl)propanoate of 3,7-dimethyl-2,6-octadienyl.

**7.** A perfuming composition or perfumed article comprising, as an active ingredient, a compound of formula

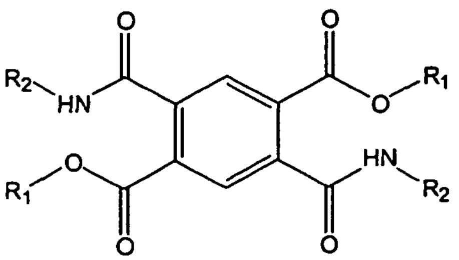

or

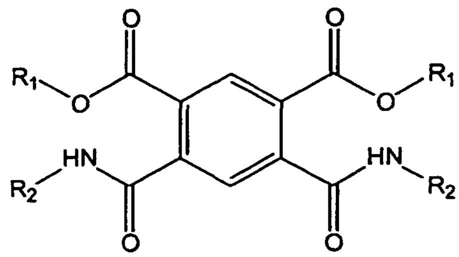

(Id)

in which the symbols $R_1$ and $R_2$ are as defined for claim 1.

**8.** A composition or an article according to claim 7, **characterised in that** the active ingredient is a compound selected from 2,5-di(octyl-carbamoyl)terephthalate of di(3,7-dimethyl-2,6-octadienyl), 1,3-di(octylcarbamoyl)isophthalate of di(3,7-dimethyl-2,6-octadienyl) or a mixture of these two compounds.

**9.** A perfumed article according to any one of claims 1 to 8, in the form of an aftershave lotion, a cosmetic preparation, a soap, a shampoo or a conditioner or another hair-care product, a shower or bath gel, a body deodorant or an air freshener, a detergent or fabric softener, or a household product.

**10.** Use of a compound as defined in any one of claims 1 to 8, as a precursor capable of liberating a perfuming alcohol.

**11.** Use according to claim 10, **characterised in that** the residue of the said compound after liberation of the perfuming alcohol is odourless.

**12.** A process for the perfuming of textiles washed in the presence of a detergent, optionally followed by treatment with a fabric softener, the process being **characterised in that** the textiles are treated in the presence of a compound as defined in claim 1.

**13.** A process according to claim 12, **characterised in that** the compound as defined in claim 1 is comprised in the detergent and/or fabric softener.

**14.** A process for intensifying or prolonging the diffusion effect of the characteristic fragrance of an odoriferous alcohol in textiles, **characterised in that** these textiles are washed in the presence of a detergent and, optionally, subsequently treated with a fabric softener, the said detergent and/or fabric softener comprising a compound as defined in claim 1.

**15.** A compound of formula (Ia) as defined in claim 3, selected from the group consisting of 2-(octylcarbamoyl)benzoate of 3,7-dimethyl-6-octenyl, 2-(dodecylcarbamoyl)benzoate of 3,7-dimethyl-2,6-octadienyl and 2-(dodecylcarbamoyl) benzoate of 3,7-dimethyl-6-octenyl.

**16.** A compound of formula (Ib) or (Ic) as defined in claim 5, selected from the group consisting of 3-(octylcarbamoyl) propanoate of 3-phenyl-2-propenyl, 4-(octylcarbamoyl)butanoate of 3,7-dimethyl-2,6-octadienyl and 3-(octylcarbamoyl) propanoate of 3,7-demethyl-2,6-octadienyl.

**17.** A compound of formula

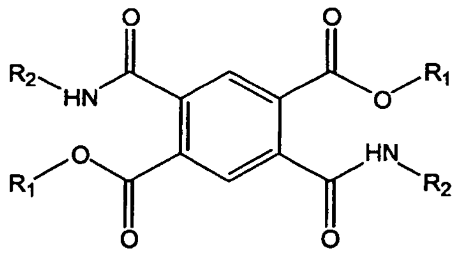

or

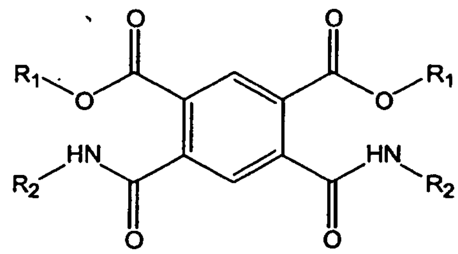

(Id)

in which the symbols $R_1$ and $R_2$ are such as defined in claim 1.

**18.** A compound according to claim 17, selected from 2,5-di(octylcarbamoyl)terephthalate of di(3,7-dimethyl-2,6-octadienyl), 1,3-di(octylcarbamoyl)isophthalate of di(3,7-dimethyl-2,6-octadienyl) or a mixture of these two compounds.

**Patentansprüche**

**1.** Duftstoffzusammensetzung oder parfümierter Artikel, enthaltend Riechstoffe, Lösungsmittel und/oder übliche zur Herstellung von Parfüms und parfümierten Artikeln verwendete Hilfsstoffe und, als Wirkstoff, eine Verbindung der Formel

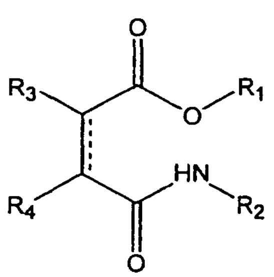

oder

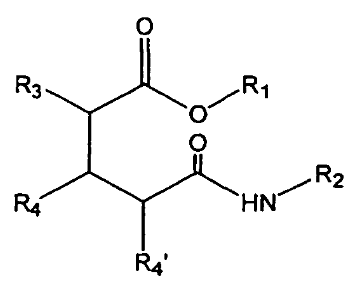

(I)

worin die punktierte Linie die Lage einer Einfach- oder Doppelbindung anzeigt; $R_1$ einen Rest darstellt, der von einem üblicherweise als Riechstoff zur Herstellung von Parfüms oder parfümierten Artikeln verwendeten Alkohol der Formel $R_1OH$ abgeleitet ist; $R_2$ ein Wasserstoffatom, einen gesättigten oder ungesättigten geradkettigen oder verzweigten $C_1$ - $C_{30}$-Kohlenwasserstoffrest oder einen aliphatischen oder aromatischen Ring mit 5 oder 6 Kohlenstoffatomen darstellt, wobei dieser Rest $R_2$ Sauerstoff-, Schwefel oder Stickstoffheteroatome tragen kann; die Symbole $R_3$, $R_4$ und $R_4$' unabhängig voneinander ein Wasserstoffatom, einen gegebenenfalls substituierten gesättigten oder ungesättigten geradkettigen oder verzweigten $C_1$- $C_{20}$-Kohlenwasserstoffrest darstellen, der ein oder mehrere Heteroatome tragen kann, oder zusammen mit den Kohlenstoffatomen, an die sie gebunden sind, aromatische oder aliphatische monozyklische, bizyklische oder trizyklische Systeme bilden können, wobei die Reste $R_3$, $R_4$ und $R_4$' funktionelle Gruppen vom Ester- und Carbamoyltyp umfassen können.

**2.** Duftstoffzusammensetzung oder parfümierter Artikel nach Anspruch 1, **dadurch gekennzeichnet, daß** der von einem Alkohol der Formel $R_1OH$ abgeleitete Rest ausgewählt ist aus Anisalkohol, Cinnamylalkohol, Fenchylalkohol, 9-Decen-1-ol, Phenethylol, Citronellol (3,7-Dimethyl-6-octen-1-ol), 3-Methyl-5-phenyl-1-pentanol, 7-p-Menthan-1-ol, Dihydromyrcenol (2,6-Dimethyloct-7-en-2-ol), alpha-Ionol, Tetrahydroionol, Geraniol, Nerol, (Z)-3-Hexen-1-ol, 1-Hexanol, 2-Hexanol, 3,3,5-Trimethylhexanol, 3,4,5,6,6-Pentamethylheptan-2-ol, 5-Ethyl-2-nonanol, cis-6-Nonenol, 6,8-Dimethyl-2-nonanol, 2,6-Nonadien-1-ol, Borneol, 1-Octen-3-ol, 4-Cyclohexyl-2-methyl-2-butanol, 6-Ethyl-3-methyl-5-octen-1-ol, 3,7-Dimethyloct-3,6-dienol, 7-Methoxy-3,7-dimethyloctan-2-ol, 2-Methyl-1-phenyl-2-propanol, 1-Phenylethanol, 2-Phenylethanol, 2-Phenylpropanol, 3-Phenylpropanol, 2-Methyl-5-phenylpentanol, 2-Methyl-4-phenylpentanol, 3-Methyl-5-phenylpentanol, Cyclomethylcitronellol, Decanol, Dihydroeugenol, 8-p-Menthanol, 3,7-Dimethyl-1-octanol, 2,6-Dimethyl-2-heptanol, Dodecanol, Octanol, Undecanol, 4-Methyl-3-decen-1-ol, Eugenol, Tetrahydro-2-isobutyl-4-methyl-4(2H)-pyranol, 2-Phenoxyethanol, Isoeugenol, Linalol, 2-Methoxy-4-propyl-1-cyclohexanol, Vanillin, Ethylvanillin, Farnesol, Cedrenol, Menthol, p-Menth-8-en-3-ol, 3,3,5-Trimethylcyclohexanol, 2,4,6-Trimethyl-3-cyclohexenylmethanol, 4-(1-Methylethyl)cyclohexylmethanol, Terpineol, Tetrahydromuguol, 3,7-Dimethyl-3-octanol, (E)-3,3-Dimethyl-5-(2',2',3'-trimethyl-3'-cyclopenten-1-yl)-4-penten-2-ol, 2,2,6-Trimethyl-alpha-propylcyclohexanpropanol, 5-(2,2,3-Trimethyl-3-cyclopentyl)-3-methylpentan-2-ol, 3-Methyl-5-(2,2,3-trimethylcyclopentyl-3-enyl)pent-4-en-2-ol, 2-Ethyl-4-(2,2,3-Trimethylcyclopentyl-3-enyl)but-2-en-1-ol, 4-(5,5,6-Trimethylbicyclo[2.2.1]hept-2-yl)cyclohexanol, 2-(2-Methylpropyl)-4-hydroxy-4-methyltetrahydropyran, 2-Cyclohexylpropanol, 2-(1,1-Dimethylethyl)-4-methylcyclohexanol, 1-(2-tert-Butylcyclohexyloxy)-2-butanol, 1-(4-isopropylcyclohexyl)ethanol und 1-(2,2,3,6-Tetramethylcyclohex-1-yl)-3-hexanol.

**3.** Duftstoffzusammensetzung oder parfümierter Artikel, enthaltend als Wirkstoff eine Verbindung der Formel

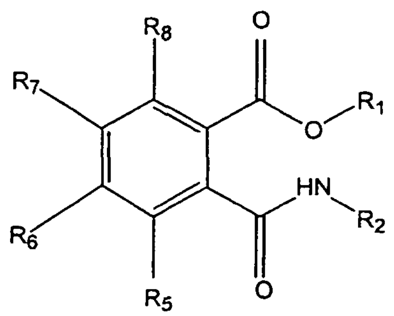

(Ia)

worin die Symbole $R_1$und $R_2$ wie in Anspruch 1 definiert sind; $R_5$, $R_6$, $R_7$ und $R_8$ unabhängig voneinander jeweils ein Wasserstoffatom oder einen geradkettigen oder verzweigten $C_1$ - $C_{20}$-Alkylrest darstellen oder jeweils zu zweien mit den Kohlenstoffatomen, an die sie gebunden sind, einen oder mehrere Ringe bilden können.

**4.** Zusammensetzung oder Artikel nach Anspruch 3, **dadurch gekennzeichnet, dass** der Wirkstoff eine Verbindung ist, die ausgewählt ist aus der Gruppe bestehen aus 3,7-Dimethyl-6-octenyl-2-(octylcarbamoyl)benzoat, 3,7-Dimethyl-2,6-octadienyl-2-(dodecylcarbamoyl)benzoat und 3,7-Dimethyl-6-octenyl-2-(dodecylcarbamoyl)benzoat.

**5.** Duftstoffzusammensetzung oder parfümierter Artikel, enthaltend als Wirkstoff eine Verbindung der Formel

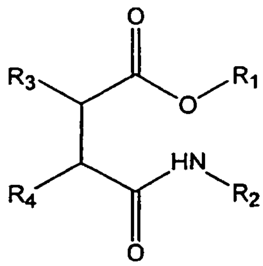

oder

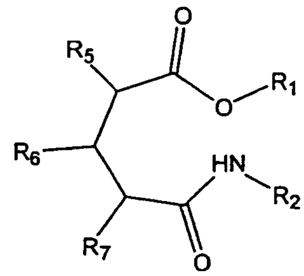

,

(Ib) (Ic)

worin die Symbole $R_1$ und $R_2$ wie in Anspruch 1 definiert sind; $R_3$, $R_4$, $R_5$, $R_6$, $R_7$ jeweils ein Wasserstoffatom oder einen geradkettigen oder verzweigten $C_1$- $C_{20}$-Alkylrest darstellen, der ein oder mehrere Heteroatome enthalten kann.

6. Zusammensetzung oder Artikel nach Anspruch 5, **dadurch gekennzeichnet, dass** der Wirkstoff eine Verbindung ist, die ausgewählt ist aus der Gruppe bestehend aus 3-Phenyl-2-propenyl-3-(octylcarbamoyl)propanoat, 3,7-Dimethyl-2,6-octadienyl-4-(octylcarbamoyl)butanoat und 3,7-Dimethyl-2,6-octadienyl-3-(octylcarbamoyl)-propanoat.

7. Duftstoffzusammensetzung oder parfümierter Artikel, enthaltend als Wirkstoff eine Verbindung der Formel

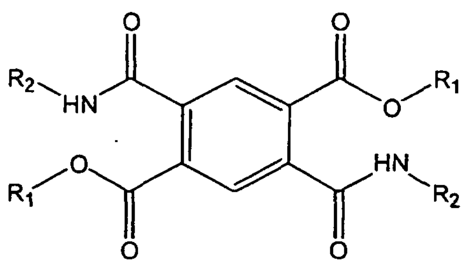

oder

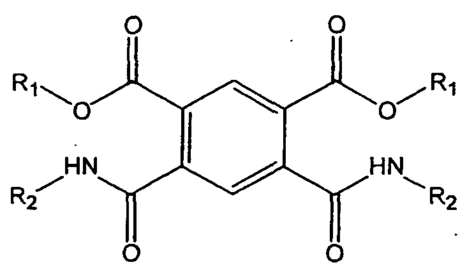

(Id)

worin die Symbole $R_1$ und $R_2$ wie in Anspruch 1 definiert sind.

8. Zusammensetzung oder Artikel nach Anspruch 7, **dadurch gekennzeichnet, dass** der Wirkstoff eine Verbindung ist, die ausgewählt ist aus Di(3,7-dimethyl-2,6-octadienyl)-2,5-di(octylcarbamoyl)terephthalat, Di(3,7-dimethyl-2,6-octadienyl)-1,3-di(octylcarbamoyl)isophthalat oder einer Mischung dieser beiden Verbindungen.

9. Parfümierter Artikel nach einem der Ansprüche 1 bis 8 in Form einer Aftershave-Lotion, eines Kosmetikpräparats, einer Seife, eines Shampoos oder After-Shampoos oder eines anderen Haarpflegeprodukts, eines Bade- oder Duschgels, eines Körperdeodorants oder eines Raumlufterfrischers, eines, Waschmittels oder Gewebeweichspülers, oder eines Pflegeprodukts.

10. Verwendung einer Verbindung gemäß einem der Ansprüche 1 bis 8 als Vorläufer, der fähig ist, einen duftenden Alkohol freizusetzen.

11. Verwendung nach Anspruch 10, **dadurch gekennzeichnet, dass** der Rest dieser Verbindung nach Freisetzung des duftenden Alkohols geruchlos ist.

12. Verfahren zur Parfümierung von Geweben, die einer Waschbehandlung in Gegenwart eines Waschmittels, fakultativ gefolgt von einer Behandlung mit einem Gewebeweichspüler, unterzogen werden, wobei das Verfahren **da-**

**durch gekennzeichnet ist, dass** die Gewebe in Gegenwart einer Verbindung gemäß Anspruch 1 behandelt werden.

**13.** Verfahren nach Anspruch 12, **dadurch gekennzeichnet, dass** die Verbindung nach Anspruch 1 in dem Waschmittel und/oder dem Weichspüler enthalten ist.

**14.** Verfahren zum Verstärken oder Verlängern der Ausbreitungswirkung des von Geweben entwickelten charakteristischen Dufts eines duftenden Alkohols, **dadurch gekennzeichnet, dass** man diese Gewebe einem Waschzyklus in Gegenwart eines Waschmittels und, fakultativ, einer anschließenden Behandlung mit einem Gewebeweichspüler unterzieht, wobei das Waschmittel und/oder der Weichspüler eine Verbindung gemäß Anspruch 1 enthalten.

**15.** Verbindung der Formel (Ia) gemäß Anspruch 3, ausgewählt aus der Gruppe bestehend aus 3,7-Dimethyl-6-octenyl-2-(octylcarbamoyl)benzoat, 3,7-Dimethyl-2,6-octadienyl-2-(dodecylcarbamoyl)benzoat und 3,7-Dimethyl-6-octenyl-2-(dodecylcarbamoyl)benzoat.

**16.** Verbindung der Formel (Ib) oder (Ic) gemäß Anspruch 5, ausgewählt aus der Gruppe bestehend aus 3-Phenyl-2-propenyl-3-(octylcarbamoyl)propanoat, 3,7-Dimethyl-2,6-octadienyl-4-(octylcarbamoyl)butanoat und 3,7-Dimethyl-2,6-octadienyl-3-(octylcarbamoyl)propanoat.

**17.** Verbindung der Formel

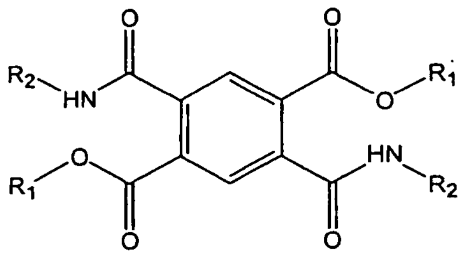

oder

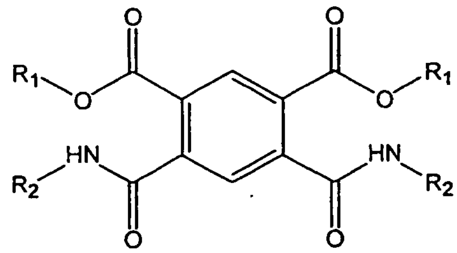

(Id)

worin die Symbole $R_1$ und $R_2$ wie in Anspruch 1 definiert sind.

**18.** Verbindung nach Anspruch 17, ausgewählt aus Di(3,7-dimethyl-2,6-octadienyl)-2,5-di(octylcarbamoyl)terephihalat, Di(3,7-dimethyl-2,6-octadienyl)-1,3-di(octylcarbamoyl)isophthalat oder einer Mischung dieser beiden Verbindungen.